# EUROPEAN PATENT SPECIFICATION

(11) **EP 2 925 236 B1**
(45) Date of publication and mention of the grant of the patent: **22.01.2020**
(21) Application number: 13795203.2
(22) Date of filing: 15.11.2013
(51) Int. Cl.: A61B 17/34, A61B 17/04, A61B 17/06, A61B 17/00

(54) **SUTURE DELIVERY SYSTEM**
NAHTFREISETZUNGSSYSTEM
SYSTÈME DE POSE DE SUTURE

(30) Priority: 28.11.2012 US 201213688005
(43) Date of publication of application: 07.10.2015
(73) Proprietor: NeoSurgical Limited, Dublin 24 (IE)
(72) Inventor: KEATING, Ronan, Dublin 24 (IE); RABBITTE, Gerard, Dublin 24 (IE); RUSSELL, Barry, Dublin 24 (IE); O'RORKE, Suzanne, Dublin 24 (IE)
(74) Representative: Hanna Moore + Curley
(86) International application number: PCT/EP2013/073989
(87) International publication number: WO 2014/082876

(56) References cited:
- WO-A1-2011/128392
- US-A- 5 527 322
- US-A- 5 948 002
- US-A1- 2004 015 177
- US-A1- 2008 208 131
- US-A1- 2010 004 665

## Description

### Field of the Invention

The present invention relates to a suture delivery system. In one configuration it relates to a suture delivery system which operably provides for deployment of an anchoring system which is usefully employed in laparoscopic surgery. In another configuration it relates to a suture delivery system which operably provides a closure system which is usefully employed in laparoscopic surgical procedures.

### Background

There are difficulties sometimes associated with the closure of the trocar wound site for example, in laparoscopic procedures. There are difficulties in particular in finding the fascia layer through which a suture must be passed to ensure good and adequate port site closure.

With deeper port sites, such as with an obese patient, it is often more difficult for the surgeon to gain deep access to the fascial layer to securely place a suture therein. In certain instances it may be necessary to cut open the wound to accurately place a suture fixation on the inner fascia layer.

The consequences of inadequate closure may be serious. For example, the patient may be subject to an early or late onset hernia, bowel stricture and/or bleeding from the port site. All of these complications have varying associated morbidities up to and including fatalities in serious undetected bowel strictures. The rate of port site herniation is widely published to be up to 3% for the normal population and double this for the obese cohort.

There are therefore a number of problems with current methods of trocar port site closure that need to be addressed, particularly for the obese patient.

There are further difficulties in anchoring or otherwise securing laparoscopic surgical devices relative to a laparoscopic surgical port, in particular with Hasson type ports. Suture stays can be difficult to manage during Hasson trocar olive fixation and can become tangled when removing or adjusting the trocar. These problems also need to be addressed in order to ensure an efficient workflow for the surgeon.

These and other problems are also found in non-laparoscopic surgical techniques where there is a desire to deliver suture to a wound site.

WO2011/128392 discloses a laparoscopic port closure device which allows for the deployment of a suture internally into an abdominal wall and the subsequent use of that deployed suture to effect a closing of the wound that was used for the port.

US5527322 discloses a suture applying device comprising a shaft having a nose piece attached at its distal end. The shaft and the nose piece are separated by a transition region, and a needle entry lumen in the shaft permits a flexible needle to be introduced in the distal direction. The needle is able to cross the transition region and penetrate tissue held therein and enter into a return lumen in the nose piece.

### Summary

The invention is defined in the appended claims.

These needs and others are addressed in accordance with the present teaching which provides a suture delivery system for deployment of a suture and anchor to enable port site closure subsequent to a laparoscopic surgical procedure. In one configuration the suture delivery system is used for anchoring purposes during a surgical procedure.

These and other features of the present teaching will be better understood with reference to the drawings which follow which are provided to assist in an understanding of the present teaching and are not to be construed as limiting in any fashion.

### Brief Description of the Drawings

The present teaching will now be described with reference to the accompanying drawings in which:
Figures 1A to 1D show examples of anchors that may be provided in accordance with the present teaching;
Figures 2A through 2H show examples of anchors that may be provided in accordance with the present teaching;
Figure 3A and 3B show examples of an anchor and cooperating driver in accordance with the present teaching.
Figure 4 is another example of a driver and anchor combination.
Figure 5 is another example of a driver and anchor combination.
Figure 6 is an example of a guide that may be used with the anchor and driver.
Figure 7 is another example of a guide that may be used with the anchor and driver.
Figure 8A is another view of the guide of Figure 7.
Figure 8B is a section through one example of a guide per the teaching of Figure 8A.
Figure 8C is a section through one example of a guide per the teaching of Figure 8A.
Figure 8D is a section through one example of another guide.
Figure 9 shows an example of how an anchor may be used to effect movement of an organ in accordance with the present teaching.
Figure 10 shows an example of how an anchor may be used to effect movement of an organ in accordance with the present teaching.
Figure 11 shows an example of a tissue grasper that may be used with the anchor of Figure 9 or 10 in accordance with the present teaching.
Figures 12A to 12E show example of anchor/driver combinations in use with other forms of guides to that described with reference to Figure 8.
Figure 13 shows an arrangement whereby a plurality of anchors can be delivered in a sequential fashion.
Figures 14A to 14E show an example of a suture guide in accordance with the present teaching and illustrate exemplary methodologies of how such a guide may be used to deliver a set of anchors.
Figure 15A shows an elevation view of an example of a suture guide provided in accordance with the present teaching, with the same guide shown in section view in Figure 15B and isometric view in Figure15C.
Figure 16A shows an elevation view of a suture guide provided in accordance with the present teaching, with the same guide shown in section view in Figure 16B and isometric view in Figure16C.
Figure 17A shows an elevation view of a guide rod provided in accordance with the present teaching, with the same guide rod shown in section view in Figure 17B and an alternate section view in Figure17C.
Figure 18A shows an elevation view of a suture guide provided in accordance with the present teaching, with the same guide shown in isometric view in Figure18B.
Figure 19A shows an elevation view of a suture guide provided in accordance with the present teaching, with the same guide shown in isometric view in Figure19B.
Figure 20A shows an elevation view of a guide rod in a deactivated configuration provided in accordance with the present teaching, with the same guide rod shown in section view in Figure 20B. Figure 20C shows an elevation view the guide rod of 20A but in an activated configuration, and again in section view in Figure 20D.
Figure 21A shows an elevation view of a guide rod provided in accordance with the present teaching in a deactivated configuration. The same guide rod is shown in section view in Figure 21B in the deactivated configuration and in the activated configuration in Figure 21C.
Figure 22A shows an elevation view of a guide rod in accordance with the present teaching provided in a deactivated configuration. The same guide rod is shown in section view in Figure 22B in the deactivated configuration and in the activated configuration in Figure 22C.
Figure 23A shows an isometric view of a guide rod with a co-operable guide in position above the guide rod, in accordance with the present teaching.
Figure 23B and C show a co-operable guide at various positions along the guide rod
Figure 23D shows an isometric view of a guide rod provided in accordance with the present teaching with a further co-operable guide in position above the guide rod
Figure 24A and 24B show a further guide rod with co-operable guide at various positions along the guide rod in isometric view, and in elevation view in Figure 24C, in accordance with the present teaching.
Figure 25A and 25B show a guide rod with cooperable guide at various positions along the guide rod in isometric view as provided in accordance with the present teaching,
Figure 26A shows an elevation view of a suture guide provided in accordance with the present teaching, with the same guide shown in section view in Figure 26B and isometric view in Figure 26C.
Figure 27A shows an elevation view of a suture guide provided in accordance with the present teaching, with the same guide shown in section view in Figure 27B and isometric view in Figure 27C.

### Detailed Description of the Drawings

The teaching of the present invention will now be described with reference to exemplary embodiments thereof which are provided to assist with an understanding of the present teaching and are not to be construed as limiting in any way. It will be appreciated that modifications can be made to the exemplary arrangements which follow without departing from the scope which is only to be limited insofar as is deemed necessary in the light of the appended claims.

Within the context of the present teaching a suture delivery system advantageously allows for the delivery of suture within an abdominal cavity of the patient. It will be appreciated that the following discussion regarding the specifics of the abdominal cavity and abdominal wall should not be construed as limiting in that a system provided in accordance with the present teaching may be used with other types of tissue including but not limited to organs, bones or the like. The use of a suture delivery system per the present teaching can be used for one or more of anchoring laparoscopic surgical equipment, assisting in the moving of internal organs to allow a surgeon access to a surgical site, or closure of a wound post completion of a surgical procedure. In such a latter configuration, where the suture is coupled to an anchor, as the suture is passed through the abdominal wall and is held within the wall by the anchors that will remain deployed within the abdominal cavity, a subsequent tightening of the sutures will cause the sides of the incision, or break in the abdominal cavity to be brought together to close the wound. The adoption of such a technique will advantageously require the use of bioabsorbable anchors, as the anchors will remain within the abdominal cavity during the healing process prior to their ultimate disintegration.

In a further embodiment the anchors may be made from a ferromagnetic material so that a magnet could be passed down a trocar and the anchors would be attracted to and adhere to the magnet, allowing them to be drawn out through the trocar. The advantage of this approach is that direct visualisation may not be necessary. However, the anchors would need to be removed prior to tying the suture in a loop. Alternatively the anchors may be themselves magnets and a ferromagnetic pick up device could be employed through the trocar to pick up the anchors.

In accordance with an aspect of the present teaching an anchor is coupled to the suture. The anchor may then be delivered to the surgical site through cooperation of the anchor with a driver tool. The driver engages with the anchor and is then used to deliver the anchor and its associated suture through to the abdominal cavity. A tensionsing of the suture will then allow for a retraction of the anchor against an inner part of the abdominal wall, where it then provides an anchoring function. In providing for delivery of the anchor, the present teaching usefully provides in one embodiment, features on one or either of the anchor or driver which serves to minimise rotation of the anchor about the needle driver during delivery. One arrangement whereby such rotation could be prevented is by making the profile of the anchor oval and engaging it with a flat on the driver. It will be appreciated that various shapes may be employed to achieve a non-rotate feature, and that the example given is not limiting. Such a non-rotate feature may be usefully employed in an application where it is required to orientate the angled cut of a driver in a given orientation.

The driver may be used in combination with a needle guide channel such as those described in our co-pending application US 13/975,599. As described therein, in use, when the driver is presented into a needle entry port it moves within the channel until such time as it meets with the anchor that is located within the channel. It then drives the anchor through the channel until it exits through an exit port where it extends into the abdominal cavity.

In this configuration, to allow the driver to pass through an arcuate needle channel, it is desirably at least partially flexible.

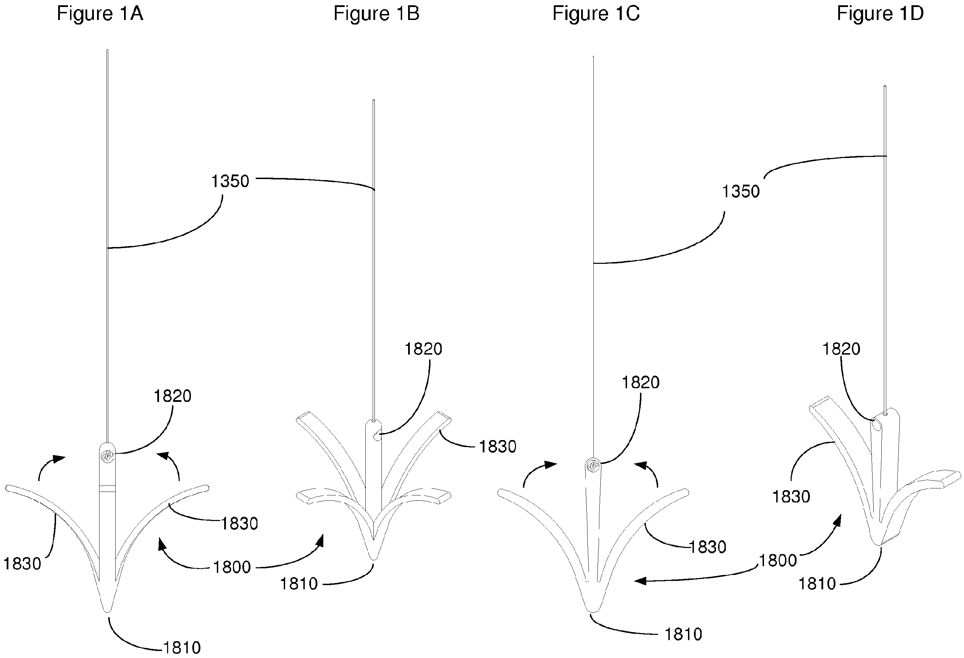

Figure 1 shows example of anchors 1800 that may be employed within the present teaching. These anchors are desirably formed of a bio-absorbable material such that they can be left within the body post-surgery. In each of the exemplary arrangements the anchors comprise a head portion 1810 and a suture coupling portion 1820 at opposing ends of the anchor. Two or more barbs 1830 are also provided. The barbs are at least partially flexible and are orientated that in the absence of an applied force thereon will extend outwardly from the head 1810. When being driven through a needle channel or the abdominal wall itself, the barbs 1830 are displaced inwardly to be substantially parallel with the major longitudinal axis of the anchor- that axis extending from the head 1810 to the suture coupling portion 1820. In this way the cross sectional area of the anchor is reduced when it is passing through the needle guide channel but once exiting from the channel the barbs will extend outwardly. On tensioning the suture, the barbs will then serve to anchor against the abdominal wall and secure the olive to the abdominal wall.

While the head portion 1810 is illustrated here with a radiused tip, this is not intended to be limiting, as the head portion may feature any number of needle tip configurations.

As detailed above, in accordance with aspects of the present teaching, the suture/anchor assembly may consist of a length of bioabsorbable suture attached to bioabsorbable anchor, provided for example in the form of a length of bioabsorbable tubing, and in one embodiment configured such that the assembly is t-shaped. The suture may be a braided suture made from a bioabsorbable polymer such as PGA for example. For fascial layer closure a USP size 0 suture is preferred. This material is ideally suited to an application where the suture maintains approximately 50% of its strength after two weeks. However it will be appreciated that the suture material may be changed depending on strength or mass loss requirements of the specific application.

In the arrangement of Figure 1, the anchor may be deployed using a driver (not shown). In such a deployment, the driver will typically engage with or couple to the suture coupling portion 1820. In one configuration the driver comprises a hollow drive tip which is receivable onto the suture coupling portion 1820- the suture coupling portion being at least partially received into the hollow tip. In this configuration the mating of the anchor with the driver provides an integrated anchor delivery tool where the head 1810 of the anchor is used to pierce the abdominal wall upon application of a force using the driver. The barbs may retract to align along an outer surface of the driver during delivery.

In such a configuration the suture 1350 may be passed internally within the driver- requiring the body of the driver to be substantially hollow. In another configuration the suture will pass along the outer surface of the driver.

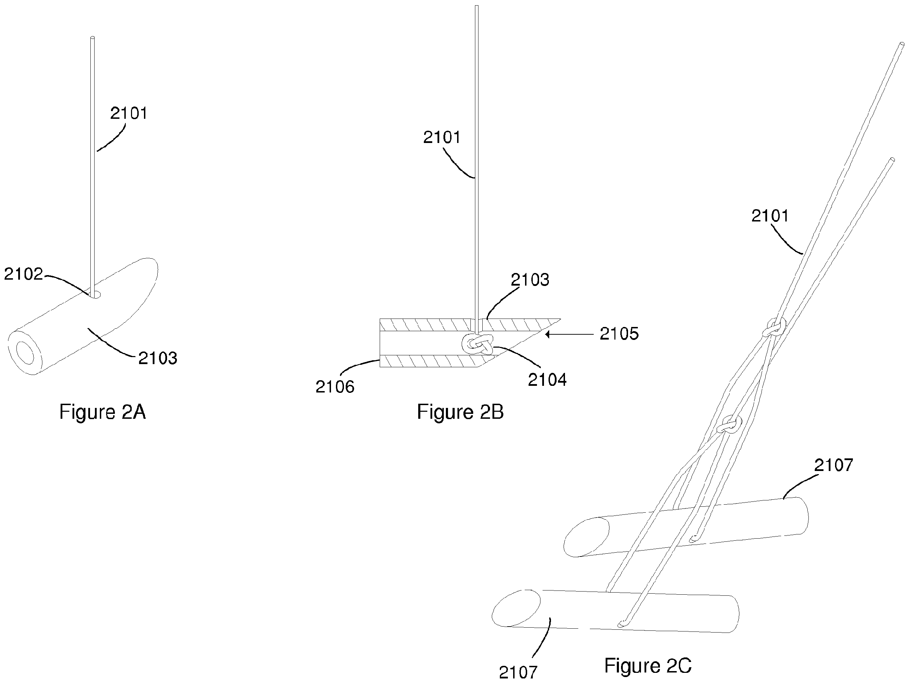

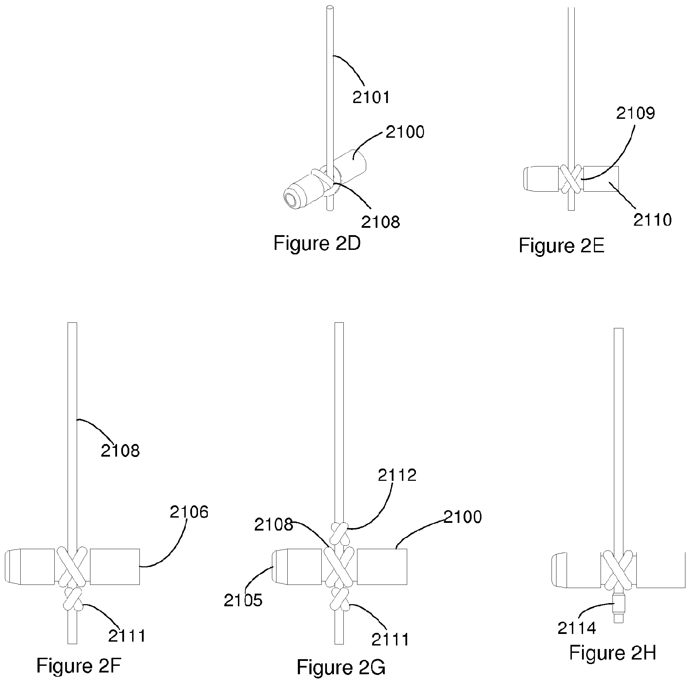

The anchor of Figure 1 is suspended at one end of the anchor from the suture 1350 so that it hangs vertically from the suture. In another configuration, suture is coupled to the anchor in a way that causes the anchor to extend laterally or horizontally from the suture. Such a configuration effectively provides a T-bar arrangement whereby the suture forms the body of the T and the anchor the horizontal member that is coupled to the body. Examples of such an arrangement will be described with reference to Figure 2.

Such an arrangement may be deployed using a braided suture which has the advantage of securely holding a knot and is well suited to the construction method illustrated in 2A and 2B. Here the anchor 2103 is formed from a short length of PGLA tubing and has a hole 2102 made in its side wall. The suture 2101 is threaded through this hole and knotted with a constrictor knot such as a double overhand knot. The knot is then pulled back into the main lumen of the tubing, the knot being too large to come through the hole 2102 on the side wall. It will be appreciated that while a double overhand knot is used in this embodiment the disclosure of such is not intended to limit the type of knot used. Additionally braided suture is not as prone to taking a shape set when wound tightly on a spool as monofilament is, making braided suture a preferred option on devices which entail the suture being wound on a spool.

In an application where anchors are not delivered through the abdominal wall, a monofilament suture could be used. Polydioxanone (PDS) and Poly(glycolideco-caprolactone) (Poliglecaprone 25) are examples of such materials. A disadvantage of monofilament suture is its reduced knot strength when compared to a comparably sized braided suture. An assembly employing monofilament suture may include a larger diameter suture, or be crimped into the anchor if the anchor is a stainless steel option. Where the suture is attached to a bioabsorbable anchor there is the option of heat welding the two components together or passing the suture through a narrow hole in the anchor and heat forming the tip of the suture, so that it does not pass back through the hole. Another option would be to place multiple barbs on the suture so that the suture itself acts as an anchor. An example of such suture will be described later.

The anchor in this configuration consists of an extruded tube. The hole in the side wall is sized to suit the suture diameter and may be disposed at the centre of the extruded length. The tubing material in an exemplary arrangement is Poly(L-lactide-co-glycolide) (PLGA) but could be made from any ratios of the following materials Poly(L-lactide-co-glycolide) (PLGA), Polylactic acid (PLA), Polyglycolide (PGA), Polydioxanone (PDS), Polycaprolactone (PCL). In one example of use, the sutures and anchors may be composed of a fast degrading polymer. In another embodiment the sutures and anchors may be composed of a slow degrading polymer. In another variant the anchors may have an additional coating of Polylactic acid (PLA) or Polycaprolactone (PCL) or a copolymer blend of these polymers in order to vary the degradation profile. In another case, the degree of crystallisation of the polymer composition of the anchors may be altered through heating and cooling treatments to change its mechanical properties.

Figure 2 shows various examples of anchors that may be deployed in accordance with the present teaching. In the arrangement of Figure 2A a suture 2101 is passed through an aperture 2102 in the body of the anchor 2103. As shown in the section view of Figure 2B, a knot 2104 may then be tied to retain the suture relative to the anchor. The anchor body 2103 is desirably hollow to allow both for location of the knot within the body, but also to allow for engagement of a driver with the anchor to enable its presentation through the abdominal wall into the abdominal cavity. A front end or leading surface 2105 is chamfered or otherwise sharpened to provide a driving surface to facilitate the passage through the abdominal wall. The back end or trailing surface 2106 is desirably the end that engages with the driver, not shown. While not shown in Figure 2B, it will be appreciated that an additional aperture may be provided in the body of the anchor whereby suture may be reintroduced into the anchor body and then travel out of the back end 2106 of the anchor. Such an implementation would be particularly useful in combination with a hollow driver whereby the suture could then pass directly into the driver. This could be particularly useful in the context of use of barbed suture where there is a desire to minimise the exposure of the barbs to the tissue until such time as deployment is required.

Figure 2C shows an alternative coupling arrangement for facilitating tethering of the anchor to suture. In this configuration the suture passes through the body 2107 of the anchor- first and second apertures are provided to facilitate this passage. On threading the suture through the body it may then be tied against itself to retain the anchor within a loop. First and second anchors could be retained against one another by passing a first anchor through the loop formed for the second. In the embodiment illustrated the body of the anchor is solid, but this is not intended to be limiting.

Figures 2D and 2E shows a further arrangement whereby suture 2101 is tied around an outer surface of an anchor 2100 in a knot 2108. The knot when formed has a knot tail. This tail desirably has a length sufficient that when tension is applied to the knot, the knot will constrict on itself prior to pulling the tail through the knot and effecting an opening of the knot. Suitable lengths of the tail are those that are related to the diameter of the suture that is used. Typical dimensions of suture are 0.25 mm and the knot tail is formed with a length of at least 2.5 mm. In accordance with the present teaching knot tails of a length at least 10-40 times the diameter of the suture used are desirable.

In this arrangement the knot is located within a recessed portion 2109 of the outer body of the anchor. In this way the physical formation of the knot does not project substantially beyond the major surface 2110 of the anchor. It will be understood that the anchor is delivered in direction that is substantially parallel to the major axis of the body and that the recessed portion prevents lateral movement of the knot as it is driven through tissue. The anchor could alternatively, or additionally, comprise a textured surface which would restrict relative movement of the knot to the anchor.

As shown in Figure 2F, the location of the knot 2108 to the anchor may be more closely secured by provision of a second knot 2111 below the anchor. Further securing could be facilitated by a third knot 2112 above the anchor such as shown in Figure 2G. Another alternative is a heat formed flat 2114 provided such as the example of Figure 2H. In an alternative embodiment, not shown, the end of the suture could be heat stamped. It will be appreciated that heat forming a braided material in this fashion will produce a flared end to the suture, which could be usefully employed to prevent the end of the suture pulling through the knot. In a further embodiment, not illustrated here, the knot or a portion of the knot could be dipped or coated in liquid bioabsorbable polymer for example polyglycolide, poly(d-lactide), poly(l-lactide), poly(dl-lactide), polycaprolactone or copolymers of those listed. Alternately the end of the suture could be dipped to form a stiff portion in a similar manner to an aglet on a shoe lace, which would prevent this portion form being pulled into the knot when tension is applied to the suture, avoiding knot failure.

Figures 2D-2H illustrate exemplary assemblies where the suture is attached to the anchor with a constrictor knot. It will be appreciated however, that other knots could be usefully employed to achieve a similar result, examples of which would be a boa knot, double overhand, strangle knot or double constrictor. This list is not intended to be limiting and the selection of knot is based on end requirements like knot security and knot profile.

The knot of Figures 2D-2E may be further secured by pre-tensioning the knot. This may be achieved by making the knot, securing one end of the suture, and to the other end applying a weight. The weight used could be in the range 0.5kg - 2kg, and more specifically 1kg for the illustrated embodiment. This range is not intended to be limiting as a number of factors influence knot security, for example the suture material, braid construction, knot selection or the presence of adjacent knots or flats as illustrated in Figures 2F-H.

In each of the above examples of Figure 2 the anchor is substantially hollow so as to allow presentation of a driver into an inner volume of the anchor. In this way the anchor will be arranged collinearly with the longitudinal axis of the driver such that a presentation of the driver through the abdominal wall- or a guide channel provided in a cooperating device- will direct the anchor in the same direction as the leading end of the driver. The driver may extend through the anchor, in which case the driver will have a piercing leading surface that will extend beyond the body of the anchor and which on delivery of the anchor to the abdominal cavity can be withdrawn from the anchor, leaving the anchor *in situ.*

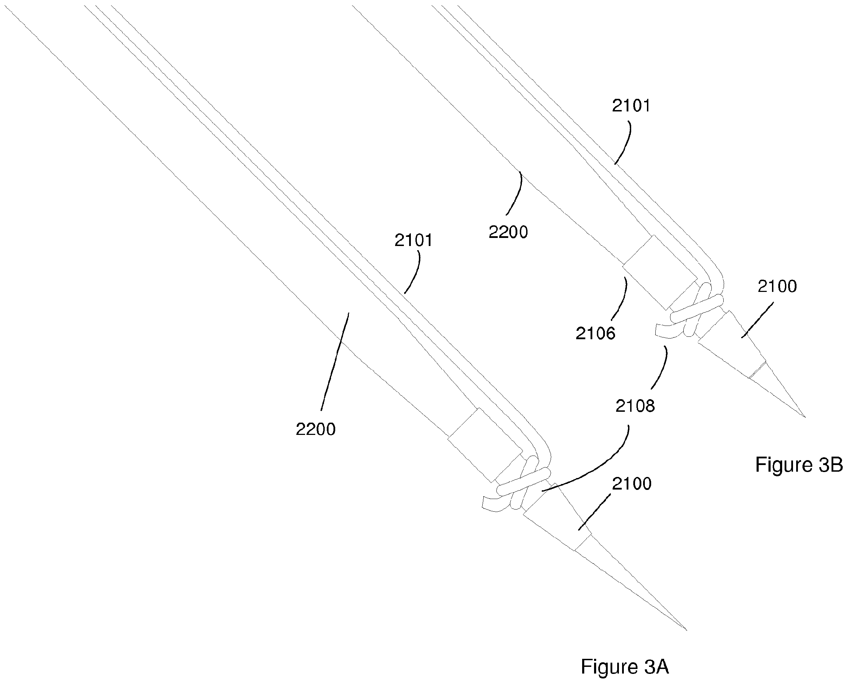

Figure 3 shows how a driver 2200 may engage with an anchor 2100- such as the examples of Figure 2 and then drive the anchor forwardly. As is shown, the driver 2200 engages with an end surface of the anchor. Pushing the driver forwardly effects a corresponding movement of the anchor through and into the abdominal cavity. In this arrangement the driver has a main body portion 2202 and a tapered end portion 2203. The tapered end portion 2203 has a smaller cross sectional diameter than the body portion 2202. In this way when the tapered end portion 2203 is presented into and engages with the end 2106 of the anchor, a step 2204 is formed. This step provides an abutment surface. When the anchor is delivered to the abdominal cavity and the driver is being withdrawn from the anchor, the abutment surface of the step 2204 will engage with or contact against the abdominal wall. This will create a resistance which facilitates the removal of the driver from the anchor. The outer diameter of the end portion 2106 in the region of the step is greater than the diameter of the tapered end portion 2203. It may extend to have a diameter as great as the diameter of the body portion 2202 of the driver.

In the arrangement of Figure 3A and 3B the driver projects through the anchor such that the anchor is located on the driver between the driver needle end portion 2205 and the main body 2202. The needle end portion is desirably sharpened to allow a piercing of the abdominal wall as necessary. The length of the needle end portion and geometry may vary- as shown in the examples of Figures 3A and 3B.

The end surface 2100 of the anchor may also include a chamfered outer surface 2207 which also facilitates the presentation of the anchor through the abdominal wall. It will be appreciated that as the driver is presented through the abdominal wall, the pressure on the leading surface 2207 will increase and will push the anchor towards the head of the driver, until such time as the inner diameter of the anchor is greater than the outer diameter of the driver, at which time movement rearwardly of the anchor is prevented.

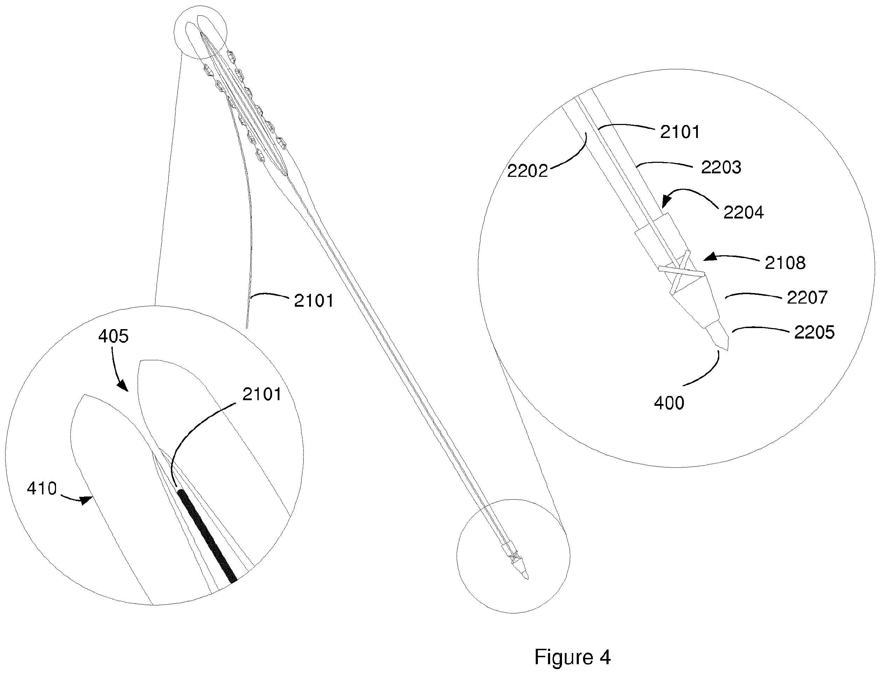

Figure 4 shows another example of an anchor and driver arrangement. The same reference numerals will be used for similar parts. Again, and similarly to that previously described, an anchor is located on a driver which is used to deliver the anchor within the abdominal cavity. In this arrangement, the needle end portion 2205 is not provided with a continuous tapered outer surface but rather is configured in a two part construct with a tip 400 being provided at the very end of the driver. The tip advantageously allows the anchor to be positioned on the non-tip portion such that the needle portion is never within the lumen of the anchor. In that scenario there would be an opportunity for tissue to lodge at the tip of the anchor and prevent accurate deployment of the anchor.

This arrangement of driver differs from that previously described in that it includes a cleat 405 or other securement feature. In this configuration the cleat is integrally formed in a head portion 410 of the driver. This is advantageous in that it allows the suture 2101 to be maintained in location along the body of the driver. It also ensures that an end portion of the suture is accessible subsequent to delivery of the anchor into the abdominal cavity. It will be appreciated that the actual location or form of the securement feature may vary. The suture may be wrapped around the head portion a number of times to attain greater security. In this embodiment, premature anchor deployment is prevented while the suture removal process remains the same in that the user pulls the suture vertically to release.

The head portion 410 also includes a textured outer surface 415 which provides improved grip to the user of the driver. This may be formed in a variety of different ways such as overmoulding an elastomeric material onto the body of the driver.

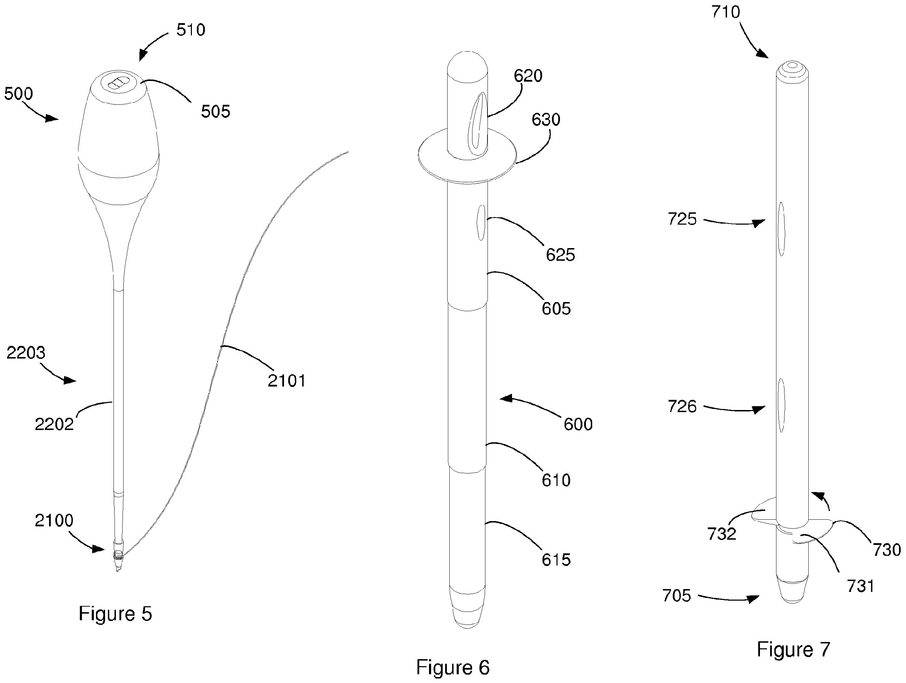

Figure 5 shows another example of a driver which again uses the same reference numerals for parts previously described. In this configuration a bulbous head portion 500 is provided. This is another grip configuration that may be advantageously employed within the context of the present teaching.

Use of such a bulbous head allows location of the driver securely into a user's palm and increases the amount of actual force that may be used in delivery of the anchor through the abdominal wall. Such an arrangement is particularly advantageous in scenarios where the driver is used by itself to effect presentation of the anchor through the abdominal wall.

In the absence of another guide structure to assist the surgeon or other operator in correctly identifying when the anchor has been delivered correctly, the present teaching provides a driver with an integrated guide indicator. An example of such an arrangement is shown in an upper surface 505 of the bulbous head 500. In this configuration a visual indicator 510 is provided. This configuration of the visual window employs three windows- although of course it will be appreciated that the dimensions or numbers of such windows may vary. The windows are colour coded and dependent on the orientation of the driver relative to the desired orientation one of the windows may be preferentially illuminated. For example, the driver may include an angular orientation sensor implemented in the form for example of an accelerometer or gyroscope, which will provide an output of the angular orientation of the driver relative to a predetermined plane. The driver may be pre-calibrated, or the surgeon may be able to calibrate the driver himself, as to a correct presentation angle. When the driver is presented to the abdominal wall at that angle- or within a predetermined range of that desired presentation angle- the window will show a first colour, for example green. When the driver orientation is slightly beyond that desired presentation angle, a second colour for example orange may be displayed. This will prompt the surgeon to modify the angle of presentation until the green is again shown. Where the angle is completely outside the preferred range a different colour, for example red, may be shown. This may prompt the surgeon to withdraw and retry. The visual indicators of green, orange and red are exemplary of the type of visual indicator that may be deployed. In addition or in replacement to such a visual indicator, the driver may include an audible warning generator which will be similarly activated dependent on the orientation of the driver relative to a desired preselected orientation. The necessary hardware and or software necessary to implement such an angular indicator may be located within the bulbous head of the driver.

Where the driver is used to directly pass the anchor through the abdominal wall it is preferable that the driver has a degree of rigidity such that it will not flex during the presentation to and through the abdominal wall. In other configuration where the driver is for example presented through a guide channel it may be necessary for all or part of the driver body to have a degree of resilience or flex to allow it adopt to the contours of the guide channel. These two forms of driver may be collectively known as rigid or flexible drivers and within the context of the present teaching it is not intended to limit to any one form of driver- except as may be deemed necessary in light of a specific application of use.

Figure 6 and Figure 7 show an example of a guide tool, or simply a guide, that may be used in combination with the driver/anchor arrangement heretofore described. In the example of Figure 6 a guide 600 is provided. The guide is configured to define a driver guide channel located between an inlet 620 and outlet 625 defined within the body of the guide 600. In this exemplary configuration the body is provided in a telescopic arrangement comprising first 605, second 610 and third 615 parts. It will be appreciated that the number and dimensions of these parts may vary dependent on the actual implementation. The telescopic arrangement facilitates different defect or trocar lengths.

In accordance with the present teaching the guide tool is co-operable with the driver and anchor. The at least one driver guide channel is dimensioned for accommodating the driver and anchor. The tool otherwise has dimensions insufficient to accommodate other surgical equipment. In this way it is different from other laparoscopic surgical equipment which may have had a dual functionality; that of providing an access port for delivery of surgical equipment into the abdominal cavity and also driver guide channels for separate delivery of suture. In accordance with the present teaching the guide tool is a dedicated sole purpose guide which allows for the delivery of suture into or through an abdominal wall.

A flange 630 is provided between the inlet 620 and outlet 625. The flange 630 provides a collar which has a larger diameter than the body of the guide 600. In use the guide body 600 will be presented through a cut formed in the abdominal wall and will pass through that cut until the collar abuts against the outer part of the fascial layer. The collar provides a locator to assist the surgeon in ensuring the relative location of the outlet 625. The driver with attached anchor may then be presented through the inlet which is located outside the body and will pass out the outlet into the abdominal wall. It will be appreciated that in certain configurations the inlet and outlet will be on the same side of the guide 600. This will require the driver to have degree of flexibility sufficient to allow it to deform to the curved channel formed between the inlet and outlet.

In another configuration, the inlet and outlet are on opposing sides of the guide 600 such that a rigid driver may be presented through the inlet and along a substantially straight channel before exiting the outlet.

In further modifications, two inlets and two outlets may be provided but it will be appreciated that the present teaching is not intended to be restricted to any specific number of inlets or outlets. For example where provided with a single inlet and outlet, the user could then rotate the device to allow use of the same inlets and outlets in another location. Furthermore multiple inlets and outlets could be used. In a further modification a single inlet could be in communication with a plurality of outlets.

Figure 7 shows another example of a guide 700 which differs in that a flange 730 is located in a lower portion of the guide 700. The guide has a leading end 705 and a trailing end 710. The flange is desirably flexible such that when the guide is being presented through the abdominal wall the flange will flex rearwardly away from the leading end 705 and attempt to conform with the contours of the body of the guide 700. To facilitate this conformity of the flange and ensure that it does not project substantially beyond the body surface during passage through the abdominal wall, it may be preferable not to form the flange as a continuous piece. In the example of Figure 7, the flange is formed from two portions 731, 732 which are located on either side of the flange body.

During the passage of the body through the abdominal wall the flange portions 731, 732 will deform in the direction shown by the arrow so as to attempt to be parallel with the major axis of the body 700. On receipt within the abdominal cavity, there is no biasing force on the flange with the result that the flange portions will return to their normal position, which is substantially perpendicular to the body- the position shown in Figure 7. In this way when a surgeon retracts the guide, the open flange will contact against an inner surface of the abdominal wall giving the surgeon tactile feedback as to the location of the guide.

This guide 700 also includes an inlet 725 and outlet 726. The inlet will, similarly to Figure 6, be located outside the body and allows for the presentation of a driver into the guide. The driver and its associated anchor will then travel out of the outlet and into the abdominal wall. Further application of pressure onto the driver will cause the driver and anchor to pass into the abdominal cavity. Retraction of the driver will then release the anchor from the driver and the application of tension onto the suture will increase the tension applied onto the anchor and bring it into contact with an inner surface of the abdominal wall. It will be appreciated that use of an anchor and guide/driver combination may be used to deliver two or more anchors. This may be usefully employed in circumstances where two or more anchors are required to effect closure of larger wound sites. For example three anchors could be deployed in a triangular configuration or four anchors in an X configuration could be deployed using the present teaching,

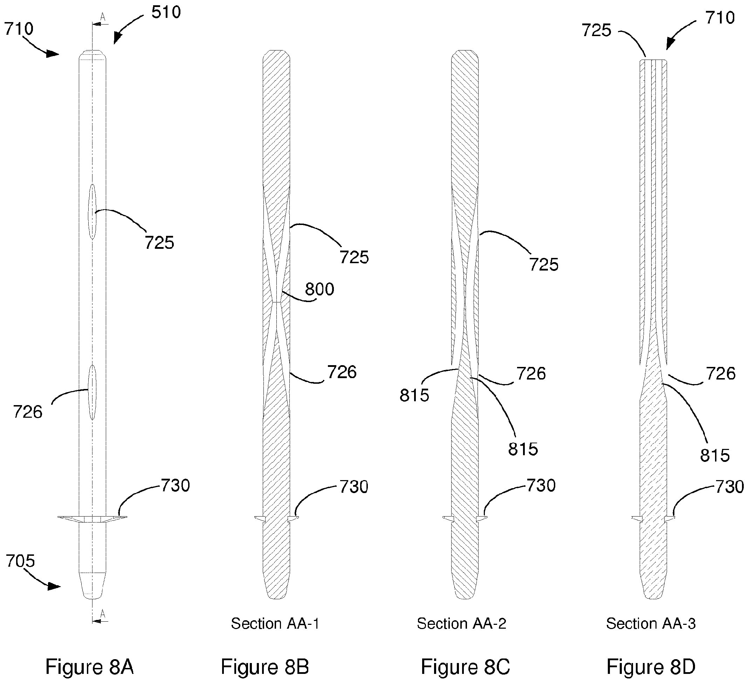

Figure 8A shows the guide of Figure 7 including a section line A-A. Figure 8B and 8C show two alternatives of guide channels that could be formed within the body of the guide.

In the example of Figure 8B, a driver will enter a guide channel 800 on a first side of the body and then exit from a second side- shown in the two examples of a solid and dash line. Effectively first and second guide channels are provided. This arrangement is particularly usefully deployed in conjunction with a rigid driver as the channel 800 is substantially straight. The angle of the channel will determine the angle of presentation of the driver and associated anchor into the abdominal wall.

In the example of Figure 8C the driver will enter and exit a guide channel 810 on the same side of the body. First and second channels 810 are provided on either side of the body and the driver will require a degree of flexibility to allow it to adopt to the curved contour of the channel 810. The driver will exit via the exit port 726 at an angle determined by the deflection surface 815 provided proximal to the exit 726.

Figure 8D shows yet a further example which is not based on the configuration of Figure 7. In this example, the entry ports are in the top of the body such that the driver will be presented coaxially with the major axis of the guide prior to being deflected outwardly by a deflection surface 815 provided proximal to the exit port 726. The guide shown in Figures 6 to 8 may include an insufflation port which may be usefully employed in maintaining pneumoperitoneum during surgical procedures.

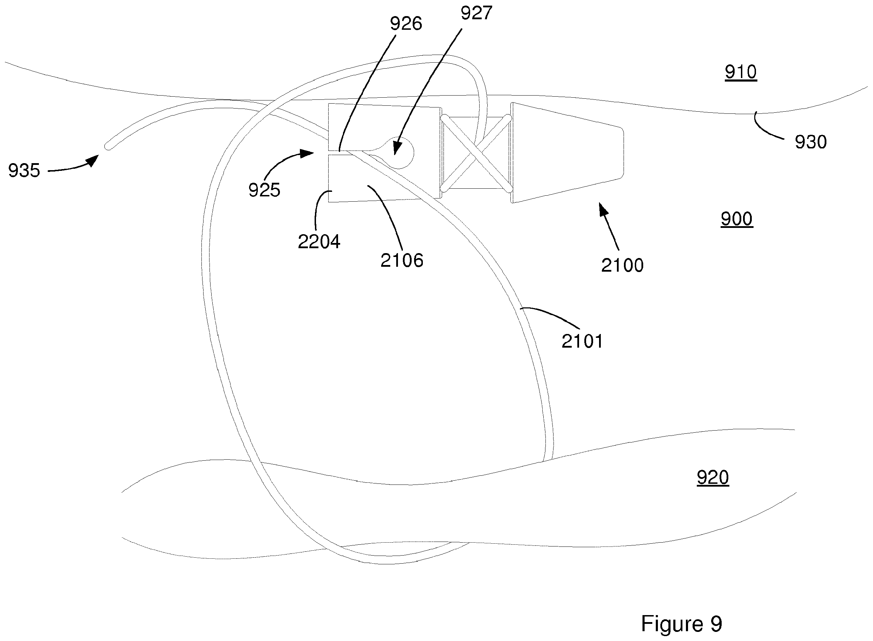

Figures 9, 10 and 11 show examples of how an anchor in accordance with the present teaching may be used to assist in the moving of internal organs to allow a surgeon access to a surgical site. The schematics of these Figures illustrate the scenario where the anchor has already been deployed into the abdominal cavity. In these examples, the void 900 represents the abdominal cavity and the abdominal wall 910 is located above same. A representative organ- the small bowel 920 - is shown as an example of the type of organ that may require movement to allow surgical access to a site otherwise occluded by its normal location.

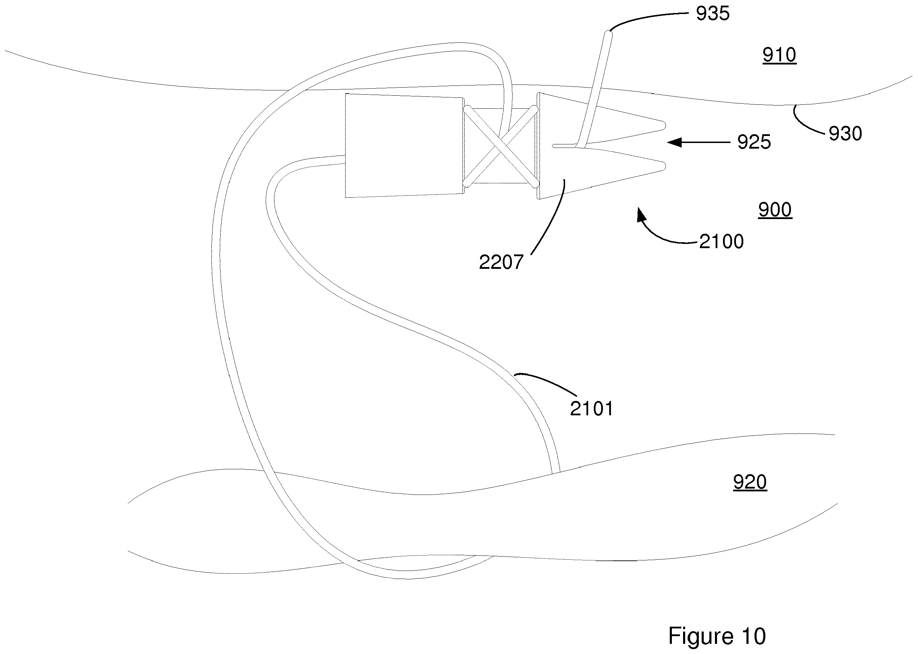

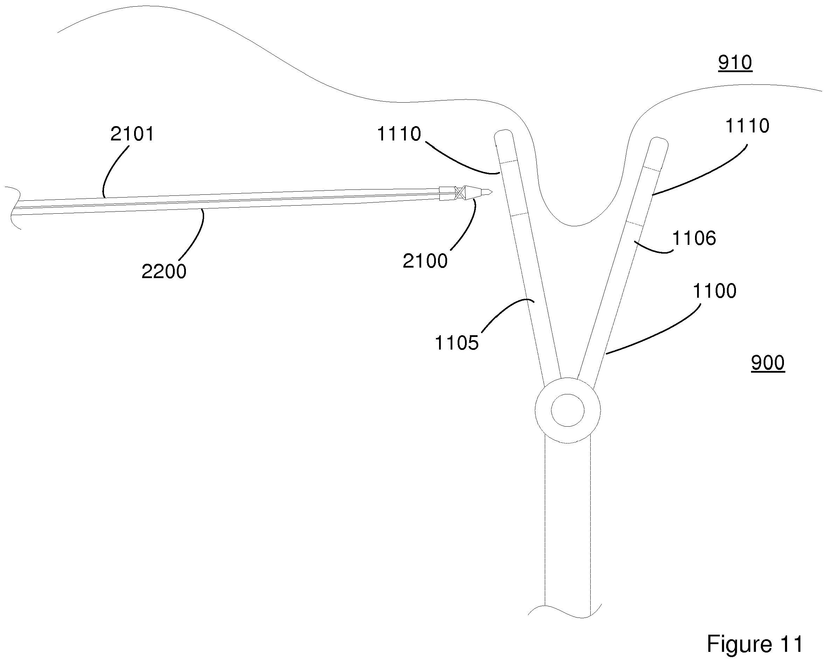

In the examples of Figures 9 and 10 an anchor 2100 is secured to suture 2101 using a knot 2108- similarly to that described before. Suture is passed through the abdominal wall- such as using the technique that will be described below with reference to Figure 11- and then looped around the organ to be moved. The suture may then be retained in the anchor using a cleat or other securement feature 925. It will be appreciated that the tensioning of the anchor against the abdominal wall 910 is achieved by tightening the suture until such time as the anchor 2100 is brought into contact with an inner surface 930 of the abdominal wall. A free end 935 of the suture may then be passed around the organ 920 and pulled so as to move the organ to a desired location. The securement of the suture within the cleat 925 maintains that desired location until such time as the surgical requirement for the movement of the organ is dispensed with, at which time the release of the suture from the cleat will allow the organ return to its normal location.

In the arrangement of Figure 9, the cleat is provided or formed in the back end 2106 of the anchor. The cleat is formed by a cut 926 in the back end that extends from the step 2204 back to an eye 927. The suture may be passed through the eye- which defines an opening in the body of the anchor sufficiently large for the suture to pass- and then locked in the cut 926 where it is retained.

In the example of Figure 10, the cleat is formed in the front end 2207 of the anchor 2100. The free end 935 of the suture is passed through the back of the anchor and through the body of the anchor where it is then secured in the cleat. Such an arrangement is advantageous as the suture cleat is adjacent to the suture and is provided in the form of a tapered slot which may provide a more secure attachment to that of Figure 9 and thereby may be usefully employed for heavier organs.

Figure 11 shows an example of how the suture shown in the abdominal wall of Figures 9 and 10 can be located. Desirably a tissue grasper 1100 which comprises two opposing arms 1105, 1106 which are moveable relative to one another is used to grasp a portion of the abdominal wall 910. Each of the arms include a window 1110 through which an anchor 2100 deployed on a driver 2200 may be presented. The anchor will therefore pass through the portion of the abdominal wall retained within the tissue grasper arms, and will bring its associated suture 2101 with it. Once through both surfaces a retraction of the driver will displace the anchor from the driver and allow for the subsequent looping of the suture about the tissue or organ that requires movement.

The grasper 1100 and driver 2200 may be activated externally of the abdominal cavity during laparoscopic surgeries.

The examples of the driver and anchor illustrated heretofore have described the suture running externally of the driver. In other configurations each of the anchor and driver may be configured to allow the suture to pass internally from the anchor through the body of the driver. The suture will then exit the driver from an exit port which is operably located externally of the body. Such an arrangement advantageously allows the surgeon to visualise the deployment of the suture and anchor as it is easier to see the suture passing relative to the exit port of the driver.

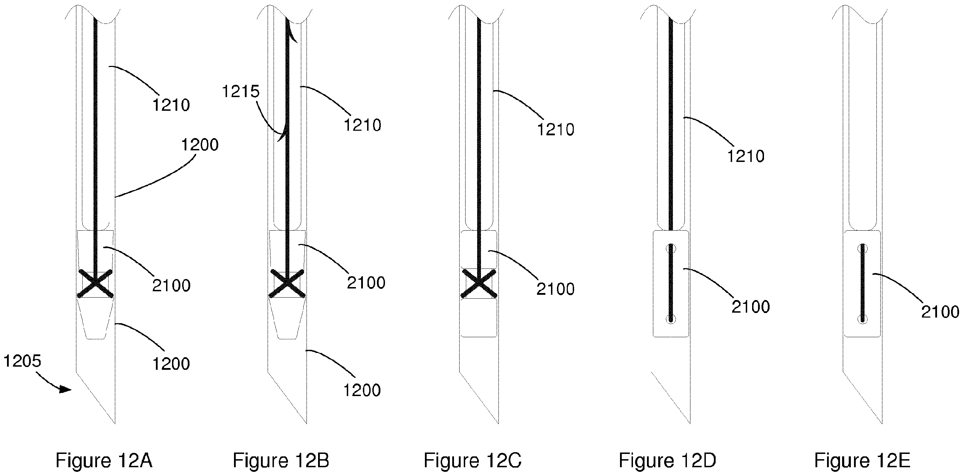

In other configurations- examples of which are described with reference to Figure 12, the anchor and driver are co-operable with a guide shaft 1200. The guide shaft 1200 is desirably provided with a tapered or sharpened leading edge. The guide shaft may be used for transporting the anchor 2100 through the abdominal wall into the abdominal cavity- the anchor being located within the guide shaft during this passage. On passage of the leading edge 1205 into the abdominal cavity, the anchor 2100 may then be biased out of the guide shaft using a driver 1210. As the driver and anchor in these configurations do not have to come into contact with the abdominal wall during the transit of the anchor into the abdominal cavity, their shapes and configurations do not have to be contoured to facilitate such passage. For this reason, anchor configurations such as those shown in Figures 12C and 12D which have blunt as opposed to tapered or sharpened ends can be used. Figure 12D also shows a variant in coupling of the suture to the anchor whereby a knot is located within the anchor as opposed to tied on the outer surface- such as Figures 12A to 12C. This arrangement of Figure 12D is similar in construct to that described with reference to Figure 2A and 2B. Figure 12E shows another variant whereby the driver is configured to receive suture within the body of the driver. In this way the suture is retained within the driver until such time as the driver is withdrawn from the surgical site, at which time the suture will be drawn out from the driver.

While the guide of Figure 12 may be used independently of another guide, it is also possible to deploy the guide in conjunction with one or more of the guides described above with reference to Figure 8. In such an implementation the anchor and driver are provided within a guide such as that shown in Figure 12. A guide such as that shown in Figure 8 is then deployed into the abdominal incision and provides a channel for delivery of the Figure 12 guide to allow deployment of the anchor.

The guide shaft of Figure 12 may also incorporate a flange or collar such as that described with reference to Figure 7, which will assist the surgeon in determination when the guide shaft has pierced the abdominal wall and is in location for delivery of the anchor into the abdominal cavity. On location the anchor can be biased out of the guide shaft using an application of pressure via the driver 1210.

In a certain configuration the length of travel of the driver 1210 within the guide shaft may be limited so as to control how far the anchor is pushed relative to the end of the guide shaft. Such control is particularly advantageous in deployment of barbed suture such as that shown in Figure 12B. In this arrangement a plurality of barbs 1215 are integrated onto the suture- examples include unidirectional and bidirectional barbs. As the barbs will naturally engage with the tissue that they contact it is important to control their deployment. In accordance with the present teaching the barbed suture may be contained within the guide shaft until such time as it is needed. The deployment may then be controlled.

An example of such control is where the driver is configured to have a length of travel just sufficient to bias the anchor out of the guide channel. In this scenario the anchor will then be hanging out of the guide, but the barbed suture will still be contained within the guide. Retraction of the guide by the surgeon will bring the anchor into contact with the abdominal wall where it will be retained. Continued retraction of the guide will then effect an exit of the barbed suture into the abdominal wall during the passage of the guide outwardly- the suture is actively deployed directly into the fascial layer. In such a configuration the barbs may advantageously be orientated opposite in direction to the anchor such that the barbs and anchor act in opposite directions to effect a securing in the fascial layers.

In the arrangements heretofore described the anchor and driver have been referenced with regard to a single deployment configuration. Effectively one anchor is provided onto the driver and then located as appropriate. The anchor is provided with a length of suture that can then be used to effect a closure of a wound or to provide an anchoring arrangement.

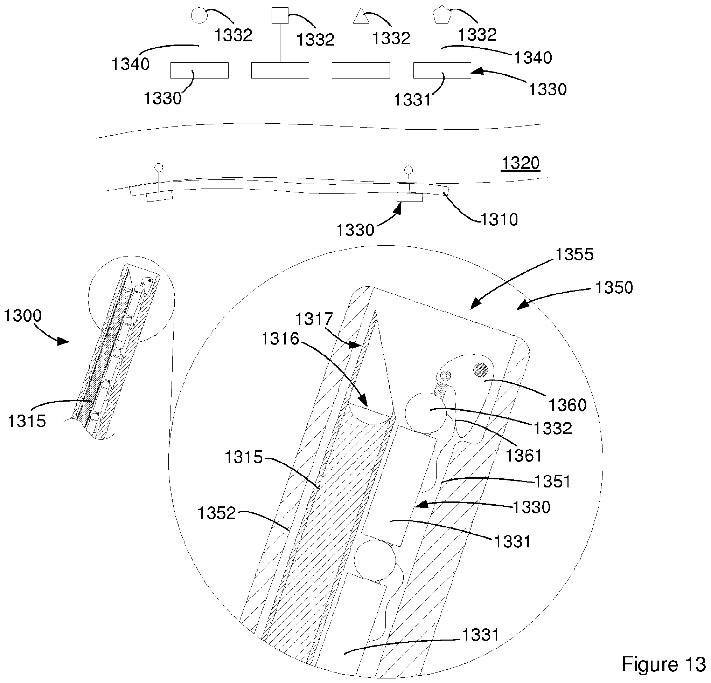

Figure 13 shows a modification where there is a desire to retain or anchor a web, mesh, or organ at a plurality of locations.

In Figure 13, the illustrated exemplary scenario is a desired retention of a web 1310 against the abdominal wall 1320. The web 1310 has an extended surface area and therefore requires retention at a plurality of locations. This is achieved in accordance with the present teaching by use of an anchor/driver combination. In this configuration an anchor 1330 is, similarly to that described before such as with reference to Figure 2, coupled at a mid-point thereof to suture 1340. It will be understood that suture is a preferred coupling mechanism but other couplers could also be considered and usefully employed. In such a configuration the anchor will hang vertically from the suture in an inverted T configuration - particularly if the point of coupling is the centre of gravity or center of mass of the anchor. In this configuration, which differs from the arrangements heretofore described, the anchor comprises first and second elements. The first element or main anchor body 1331 is similar to that described already. A second element, the anchor head 1332 is also coupled to suture but is coupled at the other end of the suture to the anchor body. In this way the suture 1340 separates the anchor head 1332 from the anchor body 1331.

Similarly to that described already, the driver 1315 is configured to engage with the anchor and to achieve a driving of the anchor to a desired location. In this arrangement, the driver comprises a socket 1316 provided in a leading end 1317 of the driver. This leading end 1317 may be sharpened or otherwise optimised to allow an at least partial penetration of the leading end of the driver into the abdominal wall or other organ as desired.

The anchor head 1332 is seatable in the socket 1316 and when seated, movement of the driver will effect a corresponding movement of the anchor 1330. In the example of Figure 13, the driver will operably effect a driving of the anchor head into the abdominal wall 1320. The length of the suture 1340 - or other coupling member- separating the anchor head from the anchor body is desirably such that the length of travel of the driver into the abdominal wall is greater than the length of the suture. In this way when the driver effects a driving of the anchor into the abdominal wall, the anchor head will be driven into the actual abdominal wall whereas the anchor body 1331 which is trailing on the suture behind the anchor head 1332 will not travel into the abdominal wall. A compressive force is then applied between the delivered anchor head 1332 and the anchor body 1331 and this will achieve retention of the anchor against the abdominal wall. By judiciously targeting the point of delivery of the anchor head, it is possible to locate a web or other material/device that requires anchoring between the anchor head and anchor body. In this way a located anchor serves to retain the web or other material/device *in situ.* Where one or more elements of this anchor are formed from bioabsorable materials these can be left in situ until such time as they dissolve. Such anchors can be used independently of other aspects of the present teaching as described herein.

In certain configurations, one anchor may be sufficient. In other configurations-such as shown in Figure 13, multiple anchors may be required. To facilitate this deployment of multiple anchors, the anchor 1330 and driver 1315 may be provided in a gun configuration 1300 whereby a plurality of anchors may be sequentially used in conjunction with the same driver 1315. By bringing a succession of anchors into contact with the same driver it is possible to use that driver to locate, in a sequential fashion, a plurality of anchors which may be spring loaded in position or ratcheted into position.

In the arrangement of Figure 13, the anchor and driver are located within the volume defined by a guide 1350. The guide comprises first 1351 and second 1352 walls and each of the anchors 1320 and driver 1315 are moveable relative to the walls of the guide.

A plurality of anchors are stacked or stackable in the guide. The anchors are desirably stacked such that the anchor head 1332 is located above the anchor body 1331. The orientation of the anchor body is desirably such that its major axis is parallel with the major axis of the guide. The head 1332 typically rests on a side surface of the anchor body 1331.

The driver is moveable between a resting and active position. In the resting position the socket 1316 is provided proximal to a head of a neighbouring anchor. The head 1332 may be displaced onto the socket 1316 by action of a pivotable actuator 1360 on the head 1332. This will typically be achieved by a triggering action effected by a user. The actuator 1360 comprises a chamfered surface 1361 which will, on movement of the actuator, displace the head 1332 into the path of the driver- where it is received and seated within the socket 1316. A second triggering action by the user will effect a movement of the driver out of the guide 1350. This is desirably achieved using a reciprocating spring motion or the like whereby the driver is driven with some force out of a mouth 1355 of the guide. As the head is seated in the socket and is also tethered to the body using the suture, this movement of the driver will effect a corresponding movement of the anchor out of the guide. The length of travel of the driver will determine the position of location of the anchor head. As discussed above, when the anchor head is located within the abdominal wall - or other desired location - a compression force is generated between the two which effects a securing of any material/device located between the two *in situ.*

The movement of a first anchor out of the guide will desirably effect a corresponding movement of the next anchor in line towards the mouth of the guide. In this way when the driver returns to its resting position, there is another anchor awaiting deployment within the gun.

The geometry of the anchor heads may vary dependent on the application. In the examples of Figure 13, anchor heads are shown being a sphere, a tetrahedron, a cube and a dodecahedron but it will be appreciated that other configurations may also usefully be employed. It will be understood that the anchor heads can be provided as a molded part as could the suture element. In other configurations the anchor head could be formed from a knot formed in the suture or other material.

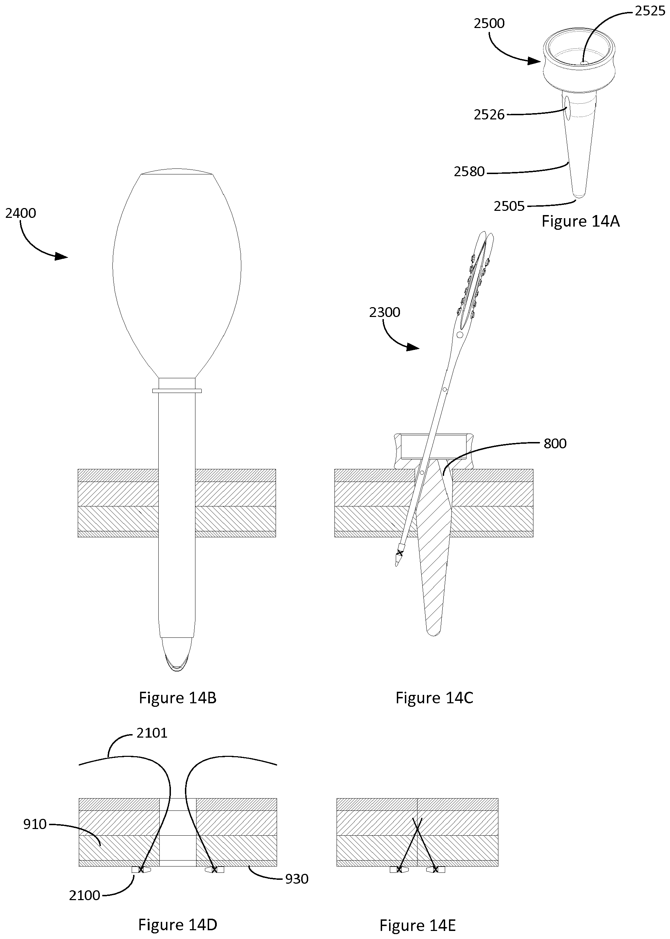

Figure 14A shows another example of a guide which may be used with a driver and anchors like those illustrated in Figure 4. A guide of this design is used by removing the Trocar 2400 shown in Figure 14B. The suture guide is positioned in the defect and a driver 2300 is used deliver an anchor 2100 though a guide channel 800 defined within the guide. This process is repeated through a second guide channel. The suture 2101 at the proximal end of the anchor in Figure 14D is pulled by the surgeon to approximate the defect and tied with a knot as illustrated in Figure 14E.

The guide illustrated in Figure 14A features a tapered leading portion 2580. In use tissue will tend to contact against the tapered portion of the suture guide. As the trajectory of the driver and outer surface of the suture guide are on divergent paths, the fascial recruitment will increase as the abdominal thickness increases. This may be disadvantageous as a larger fascial recruitment can lead to an increased likelihood of capturing nerves, leading to post operative pain.

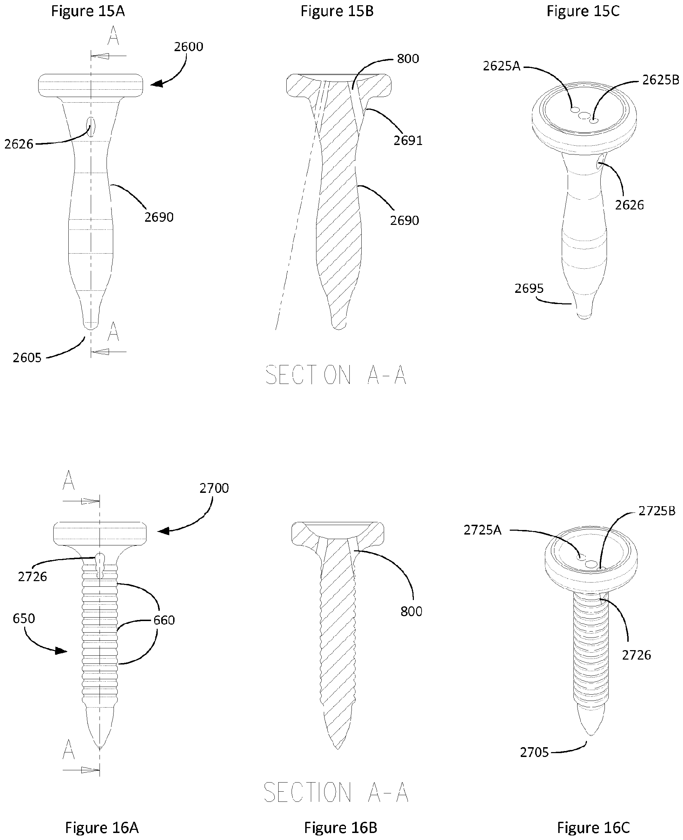

To obviate such possibilities a guide such as that shown in Figure 15 may be provided, In such a configuration, a portion of the shaft 2690 of the guide tapers as the depth increases within the specified target abdominal wall thickness 2995. This profile enables a relatively uniform fascial recruitment to be achieved across the target abdominal wall thickness.

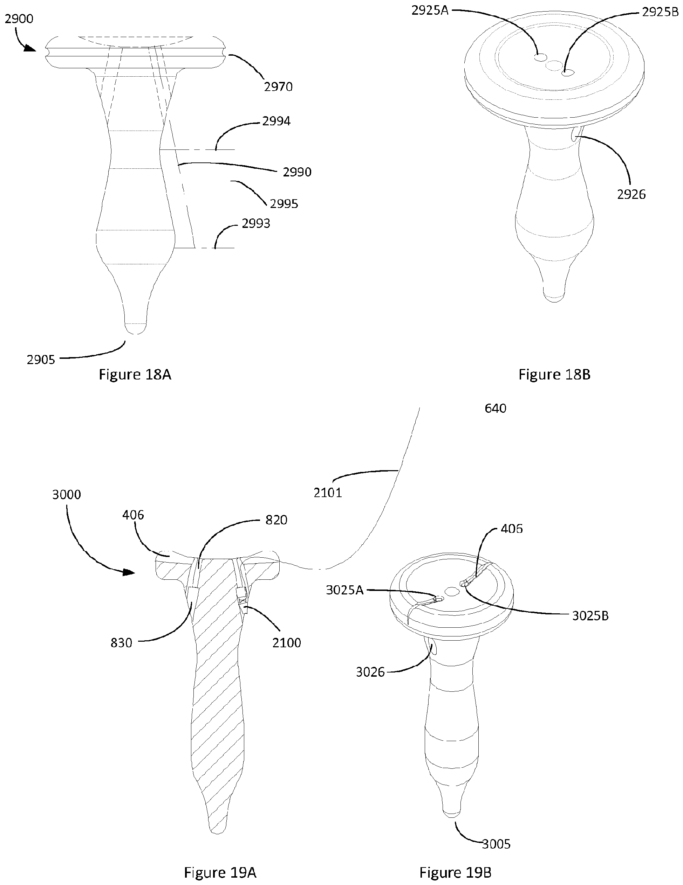

Figure 18 shows a guide which has a more exaggerated shaft geometry than that of Figure 15. Figure 18A illustrates how using such a geometry it is possible to achieve a consistent bite in the target abdominal wall thickness. The bite trajectory is illustrated by the line 2992 and the maximum and minimum abdominal wall thickness are represented respectively by lines 2993 and 2994.

Figure 18 also illustrates a grip 2970 on the head portion which may be advantageously used to aid the user in gripping the device while inserting it into a defect.

The guides of Figures 15 and 18 features a narrow tip 2695 preceded by a larger diameter section which centres the device during delivery and aids navigation through tissue layers. The guide of Figures 15 and 18 also features a tapered portion 2991, between the head portion and the shaft which facilitates sealing larger port sites.

In a further embodiment of the device of Figure 15 the device could include an exchangeable central core, not shown. This central core could form the tip portion 2695 and be substantially longer than the device of Figure 15A. This core could be passed through a trocar, and the trocar then exchanged, leaving the core *in situ* in the defect. This core would then act as a guide rail for the device similar to that of Figure 15A, but having a hollow channel though its centreline, slightly larger than the diameter of the core rod. This would be advantageous in a thicker abdominal wall, allowing for easier placement of the guide.

Figure 16A shows a further embodiment of a guide. In this embodiment the guide comprises a shaft 650 having a constant diameter along its length. There are a plurality of circumferential ribs 660 provided along the length of the shaft. These are ideally suited to prevent movement of the guide once positioned within the defect. It will be understood that the geometry of these ribs or the arrangement as either circumferential or helical may be altered to achieve varying degrees of anchoring and that the illustration shown here is not limiting.

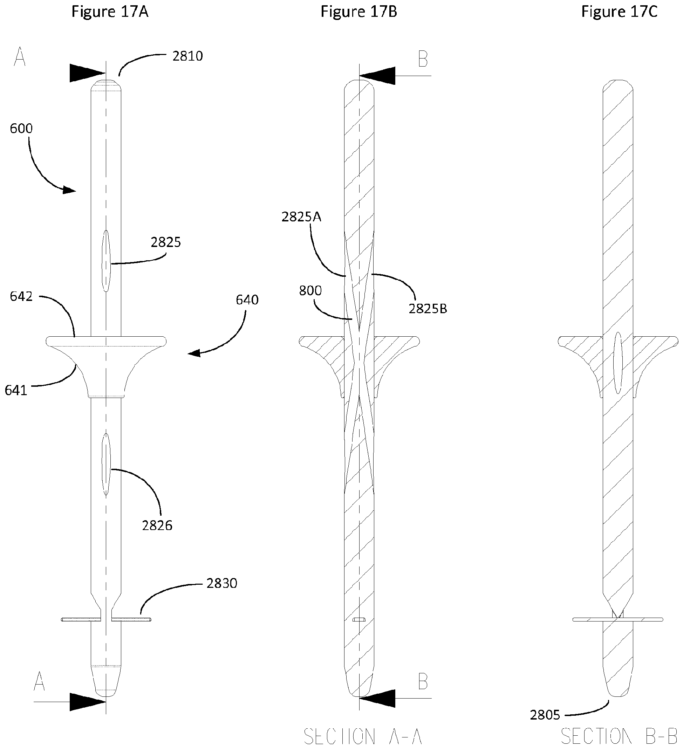

Figure 17 illustrates a guide rod. Where provided as part of a delivery system typically the guide rod 600 will be provided with a separate co operable sleeve 640. The guide rod has a deformable rectangular profile 2830 at its distal end. This portion could be configured to provide more resistance on removal than entry. In use the user would insert the guide rod through a trocar. The deformable rectangular profile would bend towards the proximal end of the guide rod as the guide rod is advanced through the trocar. Once the deformable portion exits the trocar inside the abdominal space the deformable portion adopts a t-bar configuration. The trocar is then removed. The guide rod can be positioned against the inner abdominal wall using a laparoscopic camera, or by relying on the tactile feedback of the deformable portion coming in contact with the inner abdominal wall. The sleeve 640 can then be passed over the proximal end 2810 of the guide rod and can be used to provide an airtight seal around the defect, and has the advantage of allowing the guide rod to be useable with a greater range of trocar sizes as the under surface 641 of the sleeve tapers from the guide rod diameter to a substantially larger diameter. The sleeve features an upper portion 642 which can be pressed by the user when the anchors are being deployed to stabilise the guide rod, ensuring a good vertical orientation of the rod relative to the abdominal wall. A driver and anchor, such as that shown in Figure 4, could then be used to deliver the anchor through firstly the first driver channel 2825A and secondly through the second driver channel 2825B. Upon removal of the guide rod, a pair of anchors are left in the defect as illustrated in Figure 14D and the defect can then be closed as illustrated in Figure 14E.

The deformable portion could be formed from a shape memory material such as that provided in the form of a flat Nitinol wire. The Nitinol wire could be passed through a slot in the guide rod and held in place by an interference fit. More ideally a screw could be used to secure the Nitinol. A hole for the screw could be left in the leading edge portion 2805 and provided so as to be substantially parallel to the major axis of the rod. To provide additional securement the Nitinol may have a hole at its centre with which the screw engages. The slot through which the Nitinol passes is illustrated as being substantially perpendicular to the major axis of the guide rod. This channel could be curved to bias the Nitinol in a given direction, or a dowel could be used to bias the Nitinol. A more cost effective version of the device could be manufactured from a plastic extrusion, preferably in a rectangular shape.

The guide of Figure 19 is similar to those described as Figures 15 and 18, but has two additional features. The first is a change to the driver channel. The initial portion of the driver channel 820 is of reduced diameter. In previous disclosures the driver channel diameter is governed by the diameter of the anchor which has a larger diameter than the driver. By reducing the diameter of this portion the driver has less play room in the device, which should result in more accurate delivery. The guide illustrated also features a larger diameter portion 830 to the driver channel. This portion can be utilised to receive anchors which are pre-loaded as illustrated in Figure 19A. A second feature is the inclusion of a suture retention cleat 406. This cleat can be used to hold the preloaded anchors in place by pulling the suture taut and placing the suture in the cleats as illustrated in Figure 19A.

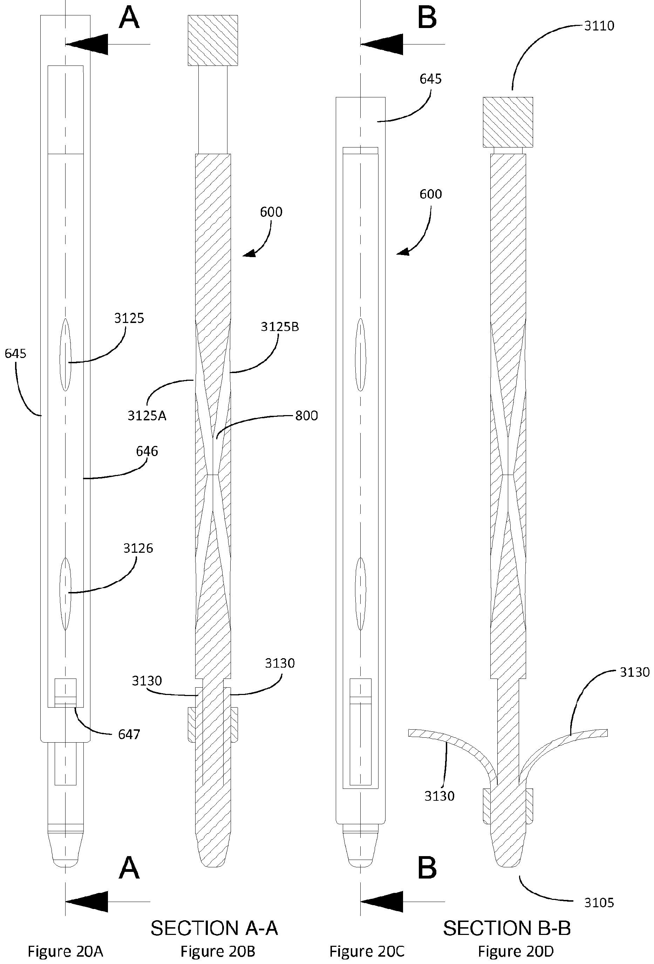

Figure 20 illustrates another arrangement of guide rod system. The guide rod features a pair of wings which are biased outwards 3130, see Figure 20D. In a non deployed state, the wings are maintained against the guide rod shaft by a sleeve 645. The sleeve features a window 646. When the device is activated by pushing the sleeve forward relative to the inner rod, this window is pushed forward, allowing the wings to deploy and form an inner abdominal wall engaging surface. The user will pull the device back until the inner abdominal wall engaging surface engages the inner abdominal wall. This activation method may also be used with the configuration shown in Figure 17. A driver and anchor of Figure 4 could then be used to deliver the anchor through firstly the first driver channel 2125A and secondly through the second driver channel 2125B. When the sleeve is retracted the edge at the distal end of the window 647 engages with the wings and forces them back into a non deployed configuration. This allows the guide rod to be removed, and upon removal a pair of anchors is left in the defect as illustrated in Figure 14D and the defect can then be closed as illustrated in Figure 14E.

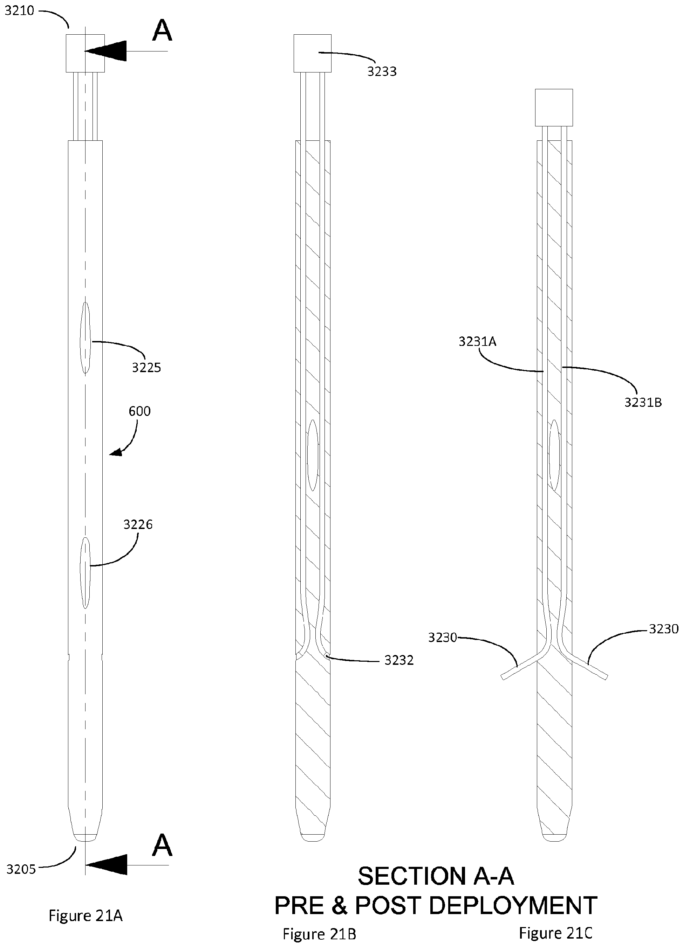

Figure 21 show a further embodiment of guide rod whereby the wings 3230 are formed from two Nitinol wires 3231 Aand 3231 B. In a non deployed configuration the Nitinol wires are in a channel in the main shaft 3232 of the guide rod. The Nitinol wires are joined to a pusher 3233. When this pusher is advanced the Nitinol wires are pushed out of the channel creating a wing feature as illustrated in Figure 21C. The device can then be used in a similar fashion to that described for the device of Figure 20.

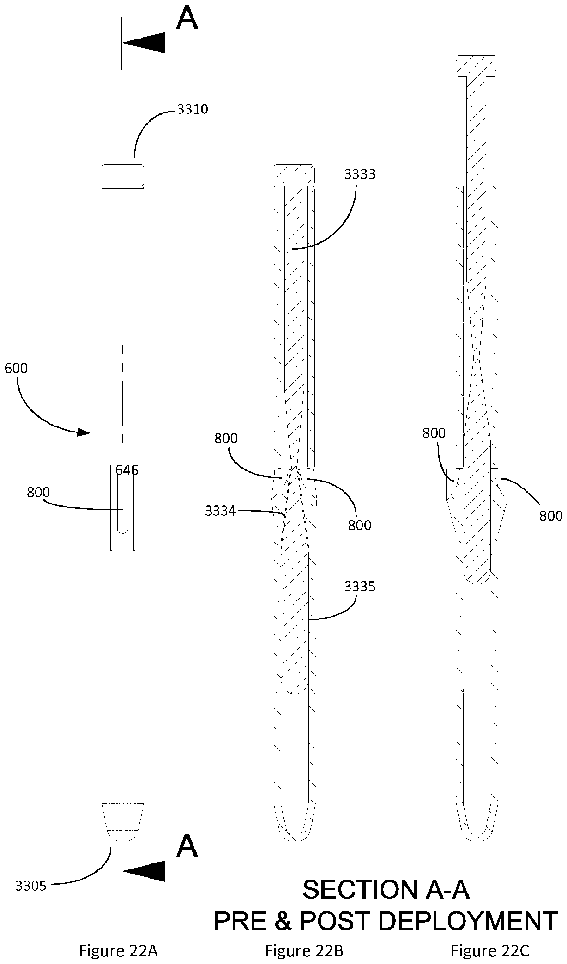

Figure 22 shows another embodiment of a guide rod. In this arrangement a pair of guide channels 800 are formed in the outer portion of the guide rod 600. Removal of the inner shaft 3333 causes the tapered surface 3334 to engage with the inner surface of the guide channel which causes the guide channel to move laterally towards a deployed configuration once the constant diameter portion 3335 of the inner shaft comes in contact with the inner surface of the guide channels. In a further embodiment, not shown the inner shaft could feature a range of constant diameter sections joined by tapering sections, This would allow the guide channels to be deployed to accommodate different diameter port sizes. A polymeric sleeve could be added to cover the guide channel portion of the guide rod, which would further increase the perimeter for use in larger defects.

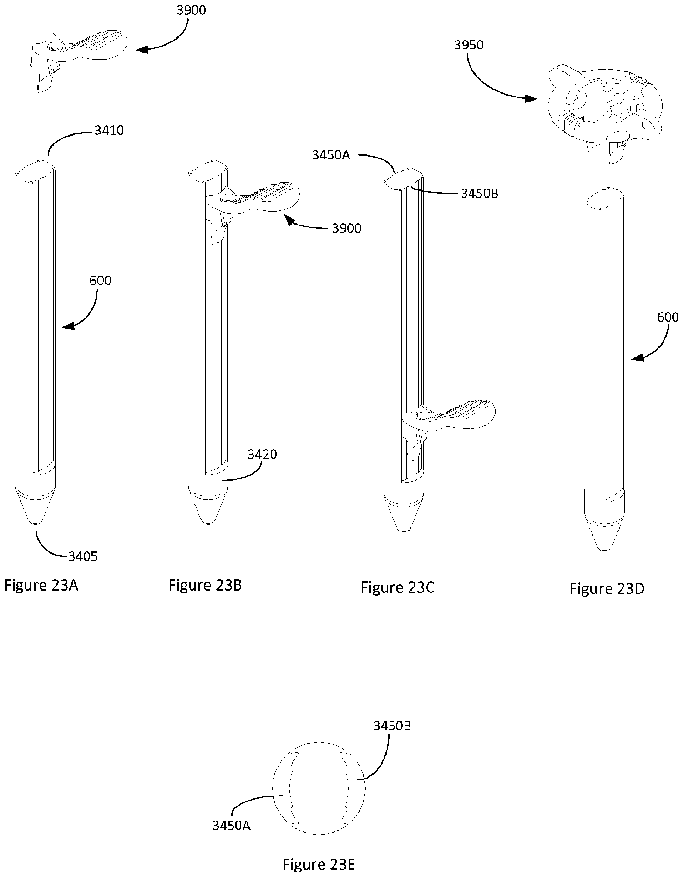

Figure 23A illustrates an isometric view of a guide rod which facilitates delivery of an guide 3900. As can be seen from the plan view of the guide rod in Figure 1E a spline 3450 is cut from the rod. This spline corresponds to the geometry of the guide 3900, which is shown over the rod in Figure 1A. The rod could be placed in the defect after the trocar is removed, or the shaft could be optimally designed to exchange through a trocar, which would advantageously maintain pneumoperitonium.

Figure 23B shows the guide 3900 interacting with the spline channel, with the guide 3900 advanced even further in Figure 23C. After advancement of the guide 3900 down the shaft on the first side of the defect and successful delivery of an anchor, the guide 3900 could be removed and placed in a similar recess on the opposite side of the rod to facilitate delivery of the guide 3900 on the second side of the defect.

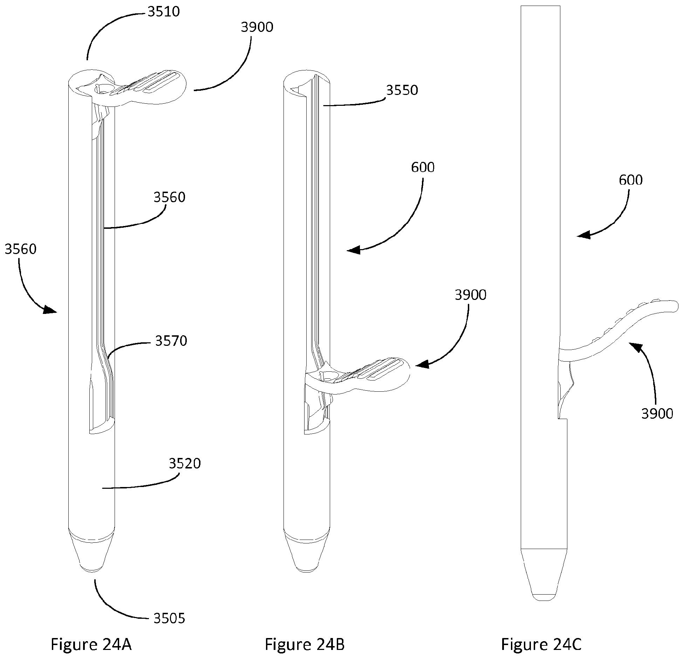

It will be understood that the round portion 3420 of the rod distal to the spline shaft is crucial to maintaining pneumoperitonium as the spline channels would present a escapement route for air. This round shaft portion could be extended in length as illustrated by 3520 in Figure 24A. The images presented here are not intended to be limiting. An additional feature which may be included, but is not illustrated, is a ratchet type of mechanism, or a catch which could hold the guide 3900 in position, once it is at the desired location.

In addition to this application this type of rod could be usefully employed to facilitate delivery of a guide 3950 as illustrated in Figure 23D.

A similar method of deployment could be achieved with a simple port exchange rod such as that provided by the Conmed Corporation under the trade name Port Saver® GhostStick.

Figure 24A and 24B show a further modification of the device illustrated in Figure 23A. In this embodiment the spline 3550 is sized such that the guide 3900 does not protrude past the outer diameter of the rod when it is presented into the spline channel of the rod, Figure 24A. The spline channel would be parallel 3560 with the rod for the majority of its travel. Towards the end of the travel the channel tapers outward 3570 to deploy the guide 3900 for use, as shown in Figures 24B and 24C. A marker, not shown, on the shaft could be placed slightly above the point at which guide 3900 protrudes past the rod diameter. In use, this marker could be coincident with the skin, ensuring that the guide 3900 is deployed below the skin, allowing for use in tighter skin incisions. This marker could be a line or band on the rod, or it could be formed by an elastomeric tube portion which could cover the distal portion of the rod.

If an elastomeric material is used, the driver and anchor could pierce the elastomeric tube during delivery, An advantage of use of an elastomeric portion is that it would increase the overall diameter of the rod portion within the defect, preventing unwanted loss of pneumoperitonium and enabling compatibility with larger defects.

In the illustrated example of Figure 24 there is a single spline channel 3550. The user would position a guide 3900 on the first side of the defect, deploy an anchor, then remove the guide 3900. The rod could then be rotated 180° to facilitate placement of an anchor on the opposite side of the defect. It will be understood that the design of the rod could be amended to include two channels, which cross paths to improve the ease of use of the rod described previously.

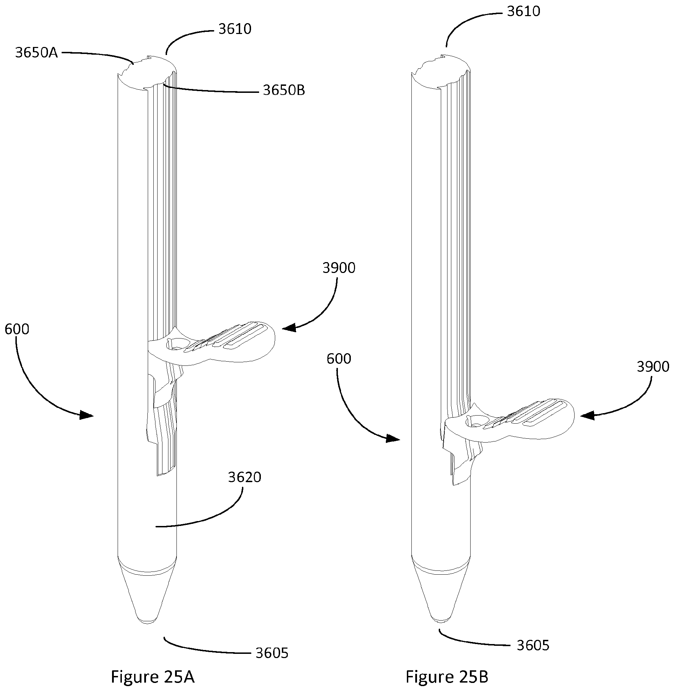

Figure 25A to 25B show a further embodiment, whereby the spline 3650 channel guides the guide 3900 through the rod where the guide 3900 exits the side of rod at a level below the skin 3680. In Figures 25A and 25B when the guide 3900 is in the first part of the guide channel, its largest profile is equal to that of the rod, allowing the profile to pass through the skin line. When advanced below the skin the guide 3900 is then guided outwards by the tapering out 3670 of the guide channels, though the side wall of the rod. The guide 3900 at this point is adjacent to the outer diameter of the rod, which maximises the fascial recruitment obtained by the driver and anchor when passed through the guide.

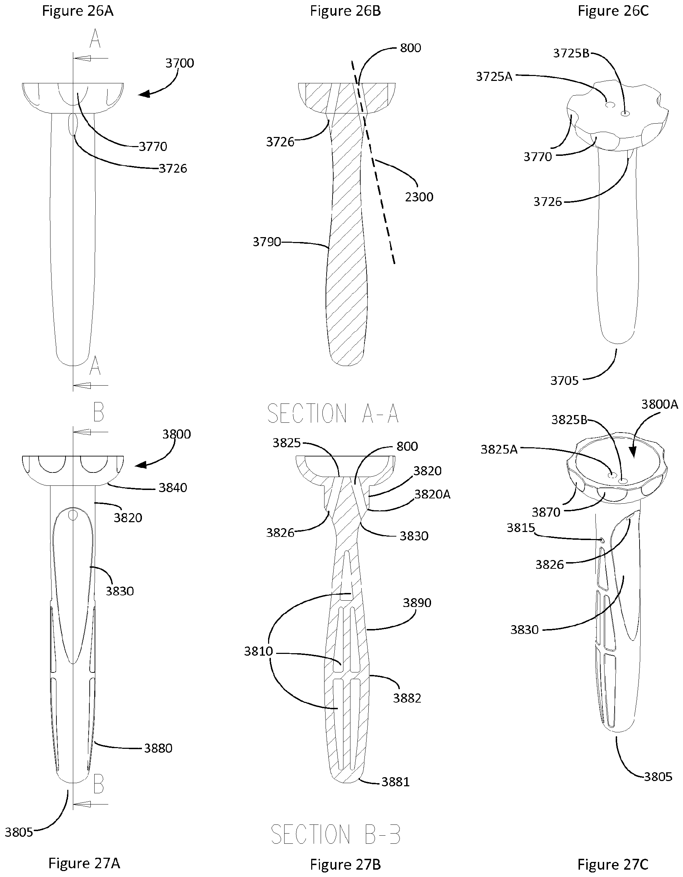

Figure 26 shows a further embodiment of the guide of Figures 15 and 18. The guide illustrated here features a series of indentations 3770 arranged circumferentially around the head portion of the guide. These indentations function as grips for the user when handling the head portion, enabling easier rotation of the device *in situ.* The size and number of such indentations could be varied to improve the grip/fell of the device. It will also be understood that a similar result could be attained by making protrusions on the outer surface. The shaft features an increasing shaft diameter portion 3790, as with the guides of Figure 15 and 18. A disadvantage of the design of Figures 15 and 18 is that the increased diameter portion may make insertion difficult.

Figure 27 shows an alternate arrangement of the guide from Figure 26. In this arrangement material in removed to leave windows 3810. The benefit of such material removal is to significantly reduce moulding times but also allows visibility through the guide. It will be appreciated by a person skilled in the art that there are many geometries that could be employed to achieve a similar result and the design presented here is not intended to be limiting. The geometry of the guide presented here has been optimised to maintain the strength of the device and minimise air escapement from an insufflated abdomen. This device is intended to be a single use device and features a pair of blind holes 3815 arranged opposite to each other. The provision of one or more blinds holes such as illustrated may be usefully employed in applications where there is a desire to make the validation of re-sterilisation process difficult. This embodiment could be configured reusable by manufacturing it from stainless steel and removing the window portions. Instead, a larger window portion could be provided in a plane perpendicular to the shown windows so that the anchors could be visualised as in this case the guide would not be translucent.

Figure 27B illustrates the cut out portion of the device which operably is in contact with the tissue adjacent to the driver and anchor trajectory. This surface ensures a consistent bite at increasing depths as illustrated earlier the embodiments of Figures 15 and 18. The profile of the embodiment of Figure 27A features a constant diameter portion 3820 close to the skin surface. This constant diameter portion 3820 desirably provides a surface that is parallel to the major axis of the guide. The guide channels 800 are defined within the body of the guide coincident with this constant diameter portion and exit the guide proximal to a lower edge 3820A of the constant diameter portion

Specifically, the guide channel comprises exit ports 3826 defined on a cutout portion 3830 below the constant diameter portion 3820. The length of the constant diameter portion is selected to be sufficient that when the device is inserted to the head portion 3840, there is no risk of the driver needle catching skin or sub dermis, which is advantageous in producing a more pleasing cosmetic effect. Another feature of the guide of Figure 27 is the curved underside surface 3840 of the head portion. This continuously curving portion can be advantageously used to prevent loss of insufflation when the device is placed in ports which are significantly larger than the constant diameter portion 3820. By providing a curved surface which extends upwardly and outwardly from a central axis of the guide, it is possible to present the guide deeper into a wound so as to maintain contact between the guide and the side walls of the wound.

The cutout portion 3830 defines a concave surface which provides an undercut below the planar surfaces defined by the constant diameter portion. When the guide is located within the abdominal wall, the fascial layer of the wall will relax into this location and ensure a good bite between the guide and the abdominal wall which improves the location of the suture and anchor through the abdominal wall.

The head portion 3800 may define a receiving well 3800A within which suture may be stored prior to deployment. While not shown in Figure 26, additional storage structures such as spigots or the like about which the suture could be wound may be provided. A lid or cap may be provided on the head portion to cover the receiving well prior to usage of the guide. In this way the suture could be safely retained within the guide until such times as the suture is required for deployment through the guide channels 800.

As shown in Figure 26, a guide provided in accordance with the present teaching may be optimised to have a length greater than the length of the driver that is used with the device. In this way when used in combination the guide provides a stop that prevents the presentation of the driver into the cavity to a depth greater than the length of the guide. This advantageously prevents the tip of the driver inadvertently contacting organs or tissue within the cavity.

The guide of Figures 26 and 27 feature a leading portion 3880 which is sized to be similar to a human finger. This leading portion should optimally have a diameter of between 1 and 2cm. The diameter of the device in Figure 27 is sized at 1cm at the leading edge 3881 and tapers up to 1.5cm at the proximal end 3882 of the leading portion which is located about the waist region of the guide, to minimise the profile of the device. This may be especially useful in a scenario where the defect is through a thick abdominal wall where the layers may have shifted laterally after removal of the trocar. Devices with narrower tips like the one illustrated in Figure 14A or 18A have a tendency to catch on layers as the device is being placed in the port site making delivery tedious. Many surgeons will pass a finger through a port before placing a trocar to check for adhesions, especially on a primary port before direct vision is established. By mimicking the human finger the tip of the devices from Figures 26 and 27 part the defect gradually over the large tip radius, whereas the smaller diameter tips Figure 14A or 18 need to be manipulated from side to side to find the opening.

It will be appreciated that guides such as those described with reference to Figures 26 and 27 allow the deployment of suture on two sides of the guide shaft. Typically the introduction of suture using such a guide will be monitored by the surgeon using a camera located within the abdominal cavity. It is not unusual in such circumstances for one side of the guide to be occluded from view. To address this problem the present teaching provides, in certain aspects a window, within the guide shaft which allows a user to view through the guide to the other side of the guide. By providing such a window, the surgeon should be able to see the driver being delivered through the device. Additionally the surgeon may find it easier to tell whether the guide is delivered below the skin surface, as this should be visible through a head portion of the guide, There are many ways for such a window to be provided, for example it the guide itself could be fabricated from a transparent material. It is not necessary for the entire guide to be formed from such a transparent material and in certain configurations only certain portions of the guide could be formed from such a transparent material.

An example of such a material which could be used with the devices of Figures 26 and 27 is a polymer such as Starex (TX-0510T). This material has a natural transparent colour when moulded with no masterbatch. The use of this material could be advantageous in a scenario where the placement of the device means camera sight of one of the delivery channels is interrupted by the guide.

In another aspect, the body of the guide could define a window by having a non-solid body form.

In another aspect the guide could be fabricated from two or more materials, with a first material being transparent and allow visibility through the guide.

The exit hole 3826 of the driver channel in the device of Figure 27 is close to approaching the true perimeter of a hole sized for the delivery channel. If we compare this to the exit hole 2526 of Figure 14A or the exit hole 3826 of Figure 26 we can observe that the perimeter of these hole are much greater than the true diameter. This is due to these holes exiting on a surface that is divergent from the channel direction. The disadvantage of such a wide exit hole is that there is greater variance were the path that the driver takes when it exits the guide. The device of 27 solves this problem by utilising the proximal cutout portion 3830 to create a surface roughly perpendicular to the driver channel path onto which the hole exits. The advantage of this is that the exit path of the driver is more consistent.

The device of Figure 27 could be used with a driver and anchor of Figure 4 to close a defect. The driver could be used to deliver the anchor through firstly the first driver channel 3825A and secondly through the second driver channel 3825B. Upon removal of the guide, a pair of anchors are left in the defect as illustrated in Figure 14D and the defect can then be closed as illustrated in Figure 14E.

While preferred arrangements have been described in an effort to assist in an understanding of the teaching of the present invention it will be appreciated that it is not intended to limit the present teaching to that described and modifications can be made without departing from the scope of the invention.

It will be appreciated that the exemplary arrangements or examples of devices have been described with reference to the Figures attached hereto. Where a feature or element is described with reference to one Figure, it will be understood that the feature or element could be used with or interchanged for features or elements described with reference to another Figure or example. The person of skill in the art, when reviewing the present teaching, will understand that it is not intended to limit the present teaching to the specifics of the illustrated exemplary arrangements as modifications can be made without departing from the scope of the present teaching.

The words comprises/comprising when used in this specification are to specify the presence of stated features, integers, steps or components but does not preclude the presence or addition of one or more other features, integers , steps, components or groups thereof.

## Claims

1. A laparoscopic delivery guide for delivery of suture and at least one anchor, the guide comprising a head (3800), a body, and a tip (3805), the body being provided between the head (3800) and the tip (3805), the guide being arranged to be inserted tip first into a wound, the head portion having a curved underside surface (3840) which extends upwardly and outwardly from a central axis of the guide such that when the guide is inserted into the wound, contact is maintained between the guide and the side walls of the wound, the guide comprising at least one guide channel (800), the guide channel (800) comprising an entry port (3825) for introduction of suture into the guide and an exit port (3826) for delivery of suture out of the guide, the entry port being defined in the head (3800) of the guide, the exit port (3826) being defined in the body of the guide,
the body comprising:
a constant diameter first portion (3820) adjacent to and joined to the head (3800) via the curved surface (3840), the constant diameter first portion having a surface that is substantially parallel to a major axis of the guide, the constant diameter first portion having the at least one guide channel (800) therein, the guide channel (800) defining a path that is not parallel to the major axis of the guide,
a cutout portion (3830) adjacent to the constant diameter first portion (3820), the cutout portion defining a concave surface providing an undercut below the surface of the constant diameter first portion,
a leading portion (3880) adjacent to the cut out portion (3830) and located between the cut out portion (3830) and the tip, the leading portion having a diameter greater than the diameter of the cut-out portion such that when the guide is located within the wound, fascial layers will relax into the cutout portion to ensure a bite between the guide and the wound which operably improves location of the suture and anchor, the exit port (3826) being proximal to an edge of the constant diameter first portion and being defined on the cut out portion (3830) below the constant diameter first portion (3820).

2. The guide of claim 1 wherein the first portion has a length of about 1 cm.

3. The guide of any one of claims 1 to 2 wherein the head defines a receiving well within which suture is stored prior to deployment.

4. The guide of claim 3 comprising a lid to cover the receiving well prior to usage of the guide.

5. The guide of any preceding claim wherein the leading portion is sized to be similar to a human finger and having a diameter of between 1 and 2cm.

6. The guide of any preceding claim wherein the body has a maximum diameter of 15 mm and a minimum diameter of 5 mm.

7. The guide of any preceding claim wherein the body has a maximum diameter of 15 mm and a second area with a diameter of 11 mm or minimum cross sectional area equivalent to that of an 11 mm circle.

8. The guide of any preceding claim wherein the body has a maximum diameter of 15 mm and a second area with a diameter of 11 mm or perimeter equivalent to that of a 13 mm circle.

9. The guide of claim 8 wherein the body has its minimum diameter at or about 2 cm below the head of the guide.

10. The guide of any preceding claim comprising a window (3810) which allows a user to view through the guide from a first side of the guide to a second side of the guide.

11. The guide of claim 10 wherein the body is formed from a transparent or translucent material thereby defining the window (3810) to allow visualisation of the second side of the guide from the first side of the guide.

12. The guide of any preceding claim being a transparent guide.

13. A laparoscopic system comprising the guide of any preceding claim and a driver, the driver being configured to cooperate with suture and anchor to effect delivery of the suture and anchor through the guide channels.

14. The system of claim 13 wherein the guide has a length greater than the length of that driver that is used with the guide, the guide providing a stop that operably prevents presentation of the driver into an abdominal cavity to a depth greater than the length of the guide.

## Patentansprüche

1. Laparoskopische Abgabeführung zur Abgabe von Nahtmaterial und mindestens einem Anker, wobei die Führung einen Kopf (3800), einen Körper und eine Spitze (3805) umfasst, wobei der Körper zwischen dem Kopf (3800) und der Spitze (3805) vorgesehen ist, wobei die Führung so angeordnet ist, dass sie mit der Spitze voran in eine Wunde eingeführt wird, wobei der Kopfabschnitt eine gekrümmte Unterseite (3840) aufweist, die sich von einer Mittelachse der Führung nach oben und außen erstreckt, sodass, wenn die Führung in die Wunde eingeführt wird, der Kontakt zwischen der Führung und den Seitenwänden der Wunde aufrechterhalten wird, wobei die Führung mindestens einen Führungskanal (800) umfasst, wobei der Führungskanal (800) eine Eintrittsöffnung (3825) zum Einführen von Nahtmaterial in die Führung und eine Austrittsöffnung (3826) zum Abgeben von Nahtmaterial aus der Führung umfasst, wobei die Eintrittsöffnung im Kopf (3800) der Führung definiert ist und die Austrittsöffnung (3826) im Körper der Führung definiert ist, wobei der Körper Folgendes umfasst:
einen ersten Abschnitt (3820) mit konstantem Durchmesser, der an den Kopf (3800) angrenzt und über die gekrümmte Oberfläche (3840) mit diesem verbunden ist, wobei der erste Abschnitt mit konstantem Durchmesser eine Oberfläche aufweist, die im Wesentlichen parallel zu einer Hauptachse der Führung ist, wobei der erste Abschnitt mit konstantem Durchmesser den mindestens einen Führungskanal (800) darin aufweist, wobei der Führungskanal (800) einen Weg definiert, der nicht parallel zu der Hauptachse der Führung ist,
einen ausgeschnittenen Abschnitt (3830) angrenzend an den ersten Abschnitt (3820) mit konstantem Durchmesser, wobei
der ausgeschnittene Abschnitt eine konkave Oberfläche definiert, die eine Kerbe unterhalb der Oberfläche des ersten Abschnitts mit konstantem Durchmesser bereitstellt, einen Führabschnitt (3880), der an den ausgeschnittenen Abschnitt (3830) angrenzt und zwischen dem ausgeschnittenen Abschnitt (3830) und der Spitze angeordnet ist, wobei der Führabschnitt einen Durchmesser aufweist, der größer als der Durchmesser des ausgeschnittenen Abschnitts ist, sodass, wenn sich die Führung innerhalb der Wunde befindet, sich Faszienschichten in den ausgeschnittenen Abschnitt entspannen, um einen Eingriff zwischen der Führung und der Wunde zu gewährleisten, was die Position des Nahtmaterials und des Ankers funktionell verbessert, wobei die Austrittsöffnung (3826) proximal zu einer Kante des ersten Abschnitts mit konstantem Durchmesser liegt und auf dem ausgeschnittenen Abschnitt (3830) unterhalb des ersten Abschnitts (3820) mit konstantem Durchmesser definiert ist.

2. Führung nach Anspruch 1, wobei der erste Abschnitt eine Länge von etwa 1 cm aufweist.

3. Führung nach einem der Ansprüche 1 bis 2, wobei der Kopf eine Aufnahmevertiefung definiert, in der das Nahtmaterial vor dem Einsatz gelagert wird.

4. Führung nach Anspruch 3, umfassend einen Deckel zum Abdecken der Aufnahmevertiefung vor der Verwendung der Führung.

5. Führung nach einem vorhergehenden Anspruch, wobei der Führabschnitt so bemessen ist, dass er einem menschlichen Finger ähnlich ist und einen Durchmesser zwischen 1 und 2 cm aufweist.

6. Führung nach einem vorhergehenden Anspruch, wobei der Körper einen maximalen Durchmesser von 15 mm und einen Mindestdurchmesser von 5 mm aufweist.

7. Führung nach einem vorhergehenden Anspruch, wobei der Körper einen maximalen Durchmesser von 15 mm und einen zweiten Bereich mit einem Durchmesser von 11 mm oder eine minimale Querschnittsfläche aufweist, die der eines 11-mm-Kreises entspricht.

8. Führung nach einem vorhergehenden Anspruch, wobei der Körper einen maximalen Durchmesser von 15 mm und einen zweiten Bereich mit einem Durchmesser von 11 mm oder einem Umfang entsprechend dem eines 13-mm-Kreises aufweist.

9. Führung nach Anspruch 8, wobei der Körper seinen minimalen Durchmesser bei oder etwa bei 2 cm unterhalb des Kopfes der Führung aufweist.

10. Führung nach einem vorhergehenden Anspruch, umfassend ein Fenster (3810), das es einem Benutzer ermöglicht, durch die Führung von einer ersten Seite der Führung auf eine zweite Seite der Führung zu schauen.

11. Führung nach Anspruch 10, wobei der Körper aus einem transparenten oder lichtdurchlässigen Material gebildet ist, wodurch das Fenster (3810) definiert wird, um die Sichtbarmachung der zweiten Seite der Führung von der ersten Seite der Führung aus zu ermöglichen.

12. Führung nach einem vorhergehenden Anspruch, die eine transparente Führung ist.

13. Laparoskopisches System, umfassend die Führung nach einem vorhergehenden Anspruch und einen Mitnehmer, wobei der Mitnehmer konfiguriert ist, um mit Nahtmaterial und Anker zusammenzuwirken, um die Abgabe des Nahtmaterials und des Ankers durch die Führungskanäle zu bewirken.

14. System nach Anspruch 13, wobei die Führung eine Länge aufweist, die größer als die Länge desjenigen Mitnehmers ist, der mit der Führung verwendet wird, wobei die Führung einen Anschlag bereitstellt, der die Präsentierung des Mitnehmers in einer Bauchhöhle bis zu einer Tiefe, die größer ist als die Länge der Führung, funktionell verhindert.

## Revendications

1. Guide de pose laparoscopique pour la pose de suture et d'au moins un ancrage, le guide comprenant une tête (3800), un corps et une pointe (3805), le corps étant prévu entre la tête (3800) et la pointe (3805), le guide étant agencé pour être introduit, la pointe en premier, dans une plaie, la partie tête ayant une surface inférieure incurvée (3840) qui s'étend vers le haut et vers l'extérieur par rapport à un axe central du guide de telle sorte que, lorsque le guide est introduit dans la plaie, un contact est maintenu entre le guide et les parois latérales de la plaie, le guide comprenant au moins un canal de guide (800), le canal de guide (800) comprenant un orifice d'entrée (3825) pour l'introduction de suture dans le guide et un orifice de sortie (3826) pour la pose de suture hors du guide, l'orifice d'entrée étant défini dans la tête (3800) du guide, l'orifice de sortie (3826) étant défini dans le corps du guide, le corps comprenant :
une première partie à diamètre constant (3820) adjacente à et reliée à la tête (3800) par l'intermédiaire de la surface incurvée (3840), la première partie à diamètre constant ayant une surface qui est sensiblement parallèle à un axe principal du guide, la première partie à diamètre constant ayant l'au moins un canal de guide (800) à l'intérieur de celle-ci, le canal de guide (800) définissant un trajet qui n'est pas parallèle à l'axe principal du guide,
une partie de découpe (3830) adjacente à la première partie à diamètre constant (3820), la partie de découpe définissant une surface concave fournissant une découpe sous la surface de la première partie à diamètre constant,
une partie avant (3880) adjacente à la partie de découpe (3830) et située entre la partie de découpe (3830) et la pointe, la partie avant ayant un diamètre plus grand que le diamètre de la partie de découpe de telle sorte que, lorsque le guide est situé à l'intérieur de la plaie, des couches de fascia se relâcheront dans la partie de découpe pour garantir une morsure entre le guide et la plaie, ce qui améliore fonctionnellement le placement de la suture et de l'ancrage, l'orifice de sortie (3826) étant proximal par rapport à un bord de la première partie à diamètre constant et étant défini sur la partie de découpe (3830) sous la première partie à diamètre constant (3820).

2. Guide selon la revendication 1, dans lequel la première partie a une longueur d'environ 1 cm.

3. Guide selon l'une quelconque des revendications 1 et 2, dans lequel la tête définit un puits de réception à l'intérieur duquel la suture est stockée avant déploiement.

4. Guide selon la revendication 3, comprenant un couvercle pour recouvrir le puits de réception avant utilisation du guide.

5. Guide selon l'une quelconque des revendications précédentes, dans lequel la partie avant est dimensionnée pour être similaire à un doigt humain et ayant un diamètre compris entre 1 et 2 cm.

6. Guide selon l'une quelconque des revendications précédentes, dans lequel le corps a un diamètre maximal de 15 mm et un diamètre minimal de 5 mm.

7. Guide selon l'une quelconque des revendications précédentes, dans lequel le corps a un diamètre maximal de 15 mm et une seconde zone avec un diamètre de 11 mm ou une zone de section transversale minimale équivalente à celle d'un cercle de 11 mm.

8. Guide selon l'une quelconque des revendications précédentes, dans lequel le corps a un diamètre maximal de 15 mm et une seconde zone avec un diamètre de 11 mm ou un périmètre équivalent à celui d'un cercle de 13 mm.

9. Guide selon la revendication 8, dans lequel le corps a son diamètre minimal égal ou environ égal à 2 cm sous la tête du guide.

10. Guide selon l'une quelconque des revendications précédentes, comprenant en outre une fenêtre (3810) qui permet à un utilisateur de voir à travers le guide d'un premier côté du guide à un second côté du guide.

11. Guide selon la revendication 10, dans lequel le corps est formé à partir d'un matériau transparent ou translucide, définissant ainsi la fenêtre (3810) pour permettre la visualisation du second côté du guide depuis le premier côté du guide.

12. Guide selon l'une quelconque des revendications précédentes, qui est un guide transparent.

13. Système laparoscopique comprenant le guide selon l'une quelconque des revendications précédentes et un élément d'entraînement, l'élément d'entraînement étant configuré pour coopérer avec la suture et l'ancrage pour effectuer la pose de la suture et de l'ancrage à travers les canaux de guide.

14. Système selon la revendication 13, dans lequel le guide a une longueur supérieure à la longueur de l'élément d'entraînement qui est utilisé avec le guide, le guide fournissant une butée qui empêche fonctionnellement une présentation de l'élément d'entraînement dans une cavité abdominale à une profondeur supérieure à la longueur du guide.
